# EUROPEAN PATENT SPECIFICATION

(11) **EP 3 413 933 B1**
(45) Date of publication and mention of the grant of the patent: **08.04.2026**
(21) Application number: 17750671.4
(22) Date of filing: 08.02.2017
(51) Int. Cl.: A61K 47/34, C08J 3/075, C08G 83/00, A61K 31/573, A61K 45/06, A61K 47/58, A61K 47/61, A61K 47/69, A61L 24/00, A61L 24/08, C08J 3/24, C09J 201/00, C08L 101/00, A61L 15/58, A61P 17/02, A61P 27/14, A61P 27/02

(54) **DENDRIMER-BIOADHESIVE POLYMER HYDROGEL NANOGLUE AND USE THEREOF**
DENDRIMER-BIOADHÄSIVER POLYMERHYDROGELNANOKLEBER UND VERWENDUNG DAVON
NANOCOLLE D'HYDROGEL DE POLYMÈRE BIOADHÉSIF DENDRIMÈRE ET SON UTILISATION

(30) Priority: 08.02.2016 US 201662292741 P
(43) Date of publication of application: 19.12.2018
(73) Proprietor: The Johns Hopkins University, Baltimore, MD 21218 (US)
(72) Inventor: RANGARAMANUJAM, Kannan, Highland MD 20777 (US); STARK, Walter, Longboat Key, FL 34228 (US); KAMBHAMPATI, Siva, Pramodh, Baltimore MD 21210 (US); SOIBERMAN, Uri, Baltimore MD 21224 (US); YIU, Samuel, Baltimore MD 21230 (US); AL-TOWERKI, Abdul-Elah, Abad, Riyadh, 11462 (SA)
(74) Representative: J A Kemp LLP
(86) International application number: PCT/US2017/016953
(87) International publication number: WO 2017/139341

(56) References cited:
- EA-A1- 200 701 306
- RU-C1- 2 093 165
- RU-C2- 2 367 525
- RU-C2- 2 521 338
- US-A1- 2011 189 291
- US-A1- 2013 136 697
- XIANGDONG BI ET AL: "Synthesis of PAMAM dendrimer-based fast cross-linking hydrogel for biofabrication", JOURNAL OF BIOMATERIALS SCIENCE. POLYMER EDITION., vol. 26, no. 11, 23 June 2015 (2015-06-23), NL, pages 669 - 682, XP055622252, ISSN: 0920-5063, DOI: 10.1080/09205063.2015.1056716
- MARK W. GRINSTAFF: "Dendritic macromers for hydrogel formation: Tailored materials for ophthalmic, orthopedic, and biotech applications", JOURNAL OF POLYMER SCIENCE PART A: POLYMER CHEMISTRY, vol. 46, no. 2, 5 December 2007 (2007-12-05), pages 383 - 400, XP055128002, ISSN: 0887-624X, DOI: 10.1002/pola.22525
- RYAN J. SEELBACH ET AL: "Multivalent dendrimers presenting spatially controlled clusters of binding epitopes in thermoresponsive hyaluronan hydrogels", ACTA BIOMATERIALIA, vol. 10, no. 10, 1 October 2014 (2014-10-01), pages 4340 - 4350, XP055199858, ISSN: 1742-7061, DOI: 10.1016/j.actbio.2014.06.028
- BRAVO-OSUNA I ET AL: "Interfacial Interaction between Transmembrane Ocular Mucins and Adhesive Polymers and Dendrimers Analyzed by Surface Plasmon Resonance", PHARMACEUTICAL RESEARCH, KLUWER ACADEMIC PUBLISHERS-PLENUM PUBLISHERS, NL, vol. 29, no. 8, 8 May 2012 (2012-05-08), pages 2329 - 2340, XP035087064, ISSN: 1573-904X, DOI: 10.1007/S11095-012-0761-1
- ABIGAIL M. OELKER ET AL: "Ophthalmic adhesives: a materials chemistry perspective", JOURNAL OF MATERIALS CHEMISTRY, vol. 18, no. 22, 1 January 2008 (2008-01-01), pages 2521, XP055041676, ISSN: 0959-9428, DOI: 10.1039/b719791h
- VANDAMME TH.F. ET AL.: "Poly(amidoamine) dendrimers as ophthalmic vehicles for ocular delivery of pilocarpine nitrate and tropicamide", JOURNAL OF CONTROLLED RELEASE, vol. 102, 2005, pages 23 - 38, XP027664499

## Description

### FIELD OF THE INVENTION

The present invention relates to the field of hydrogel adhesives, and more particularly, to wound repair and treatment of disorders.

### BACKGROUND OF THE INVENTION

Repair of wounds after traumatic or surgical injury is of significant clinical importance. These wounds are often sealed with sutures. However, sutures have a number of disadvantages, including invasive procedures, uneven healing, propensity to become loose and/or broken, and often requiring removal by a skilled practitioner. Furthermore, suturing may cause inflammation, increase the risk for infection and, in the case of corneal wounds, may lead to neovascularization and induce astigmatism. In comparison, sutureless surgeries have a lower rate of infection compared to ones with sutures (Stonecipher K, et al., Arch Ophthalmol., 109(11):1562-1563 (1991)). Previous studies have explored hydrogel adhesives, but they are disfavored due to lack of biocompatibility, lack of tensile strength, and limited sites for application (Grinstaff MW, et al., Biomaterials, 28(35):5205-5214 (2007); Grinstaff MW, et al., Chemistry-A European Journal, 8(13):2838-2846 (2002)). Grinstaff MW, et al., Journal of Polymer Science part A: Polymer chemistry, 46(2):383-400 (2007) describes dendritic macromers for hydrogel formation and tailored materials for ophthalmic, orthopedic, and biotech applications. US 2011/189291 A1 describes dendrimer hydrogels.

Preventing and combating inflammation, infection, and other complications would facilitate the healing of tissue. This would be even better if one could use the system for the delivery of one or more active agents. In the treatment of ocular disorders, topical antibiotics and steroids reduce the risk of infection and corneal scarring, respectively. However, the drugs are often cleared through tear secretions, and therefore repeated dosing is required which may lead to patient non-compliance, discoloration of cornea, elevated intraocular pressure, and corneal irritation and toxicity (Gibson JM, et al., US Ophthalmic Review, 8(1):2-7 (2015); Mahajan HS, et al., Carbohydrate Polymers, 122:243-247 (2015)).

Therefore, it is an object of the present invention to provide a nanoglue or its precursor formulation to effectively seal tissues (e.g., good coverage, biocompatibility, mechanical strength, and degradability) and promote wound repair.

It is another object of the present invention to provide a composition for use in a method of sealing tissue using a hydrogel or its precursor that can be triggered to gelate and seal the tissue upon application of external stimuli by a practitioner or upon introduction to the tissue.

It is yet another object of the present invention to provide a composition for use in a method for sealing ocular tissue, and more preferably for simultaneously delivering active agents locally in a sustained manner.

It is yet another object of the present invention to provide a composition for use in a method of treating corneal inflammation and infections by delivering drugs such as corticosteroids (anti-inflammatory drugs) and antibacterial drugs in a sustained manner using an injectable gel formulation that can be administered subconjunctivally.

### SUMMARY OF THE INVENTION

The present invention provides a composition comprising:
generation 2-10 poly(amidoamine) (PAMAM) dendrimers comprising one or more terminal hydroxyl groups and one or more photo-crosslinkable groups;
bioadhesive polymers comprising one or more photo-crosslinkable groups; and
a photoinitiator;
wherein the dendrimers and the bioadhesive polymers are crosslinked to each other through their respective photo-crosslinkable groups upon exposure to one or more external stimuli to form a nanoglue within a tissue, and
wherein the external stimuli is ultraviolet radiation, cobalt blue light, argon laser, or visible light.

The present invention also provides a composition for use in a method of sealing tissue in an individual in need thereof, the method comprising:
administering to a tissue of the individual in need thereof the composition of the present invention; and
crosslinking the dendrimers and bioadhesive polymers within the tissue by exposure to the one or more external stimuli to form a nanoglue,
wherein the external stimuli is ultraviolet radiation, cobalt blue light, argon laser, or visible light.

The present invention also provides a kit comprising a dosage unit of the composition of the present invention.

The present invention also provides a composition of the present invention, for use in a method of therapy or surgery.

The present invention also provides a composition of the present invention, for use in healing an eye of an individual in need thereof, wherein upon use, the dendrimers and bioadhesive polymers are photo-crosslinked within the eye of the individual.

A hydrogel or hydrogel precursor composition for sealing tissue and optionally delivering therapeutic, prophylactic, and/or diagnostic agents has been developed and is referred to herein as a "nanoglue". The nanoglue is formed with one or more dendrimer molecules (or derivatives thereof) and one or more bioadhesive polymers (or derivatives thereof), wherein the dendrimer molecules and the bioadhesive polymers are crosslinked upon application of one or more external stimuli within the tissue.

The dendrimers and the bioadhesive polymers are chemically modified to contain photo-crosslinkable groups (e.g., thiol groups and vinyl groups; thiol groups and maleimide groups; thiol groups and alkene groups; thiol groups and alkyne groups; amine groups and aldehyde groups; amine groups and carboxylate groups; methacrylate groups; and acrylate groups). These dendrimers and bioadhesive polymers with functional groups are cured to form the nanoglue *in situ* at tissue sites after exposure to external stimuli selected from ultraviolet irradiation, cobalt blue light, argon laser or visible light. Optionally, the therapeutic, prophylactic, or diagnostic agent is conjugated to or complexed with a dendrimer molecule, and is released at the administered sites in a sustained and controlled manner.

In certain embodiments, the one or more bioadhesive polymers are hyaluronic acid, chitosan, alginate, agarose, carboxymethylcellulose, hydroxymethylcellulose, methylcellulose, cellulose, polyalkylene oxide, poly(acrylic acid), poly(hydroxyethyl methacrylate), poly(vinyl pyrrolidone), poly(vinyl alcohol), or derivatives thereof.

Dendrimer nanoglues have been developed which both seal and provide for controlled, sustained local release of therapeutic, prophylactic and/or diagnostic agent. The nanoglue has desirable mechanical properties to hold the tissue. It also can be used to deliver agents accelerating wound healing and delivering antibacterial and anti-inflammatory drugs to prevent infection and scar tissue formation. The formulation is surgeon friendly. It is a viscous liquid during application and polymerizes rapidly upon laser illumination, providing a longer time window for surgeons to work with. The strength of the nanoglue can be modulated by the duration of laser application or by altering the percentages of individual components of the nanoglue.

Other materials can be incorporated into the formulations to increase flexibility, strength or control drug delivery properties. An example is hyaluronic acid to increase strength and flexibility. Therapeutic, prophylactic and/or diagnostic agents can be incorporated directly, bound to the dendrimers, or formulated into particles, to enable sustained release of therapeutic agents such as steroids and antibacterial drugs (antibiotic drugs). This can be used to reduce eye drop usage, thereby reducing side-effects.

The formulation has multimodal applications and can be modified for administration for different locations or purposes, for example, as a looser gel for subconjunctival administration to increase drug availability and aid sustained drug release for various corneal and anterior segment diseases such as corneal inflammation, corneal neovascularization, corneal graft rejection, and possibly iritis and anterior uveitis. The percentage of individual components is modified with appropriate dilution to form transparent flexible drug depot gels that can be injected intravitreally for sustained release of drugs for a variety of posterior segment diseases such as diabetic retinopathy, choroidal neovascularization (CNV) secondary to age related macular degeneration (AMD) and other retinal pathologies. Using hyaluronic acid as the major component in the modified formulation integrates well with the vitreous gel, causing dendrimer with drugs to release from the gel and delivering drugs to targeted retinal cells for enhanced and long-term efficacy resulting in significant decreased frequency in intravitreal injections.

The formulation provides advantages for treatment of conditions requiring frequent eye drops that often cause ocular surface irritation and toxicity and may have a better safety profile in terms of intraocular pressure (IOP) and patient discomfort. The subconjunctival gels have reduced side effects as compared to eye drops. The photocrosslinkable dendrimer-hyaluronic acid based nanoglues are useful in sealing corneal incisions and simultaneously releasing antibiotic/steroids for prevention of infection and inflammation and to accelerate corneal wound healing. In addition to the benefits of being able to be applied *in situ* and photo-cured in a tailored manner to provide a high crosslink density through the use of the dendrimer; the inclusion of hyaluronic acid in the gel promotes wound healing and integrates into corneal stroma; while simultaneously releasing antibiotics/steroids to address infections/inflammation in a sustained manner. The formulation is also transparent, which is clearly beneficial in ocular applications.

In preferred embodiments, the nanoglue is for use in a method wherein it is administered to seal corneal tissue and/or administered to treat various corneal and anterior segment disorders such as corneal inflammation, corneal neovascularization, and complications with corneal graft procedures, iritis, and uveitis. The nanoglue improves the healing of corneal incision, compared to that treated with sutures or non-treated. It also withstands the high intraocular pressure of the wounded eye compartments, avoids leakage, adheres strongly to the cornea, and results in less corneal edema or fibrosis, compared to sutures. The healing of the cornea is more rapid and has less scarring, inflammation, and neovascularization compared to treatment with sutures.

In certain embodiments, the therapeutic agents are anti-inflammatory, anti-infectious, or anti-angiogenesis small molecules or biomacromolecules.

A benefit of the nanoglue is the targeted biodistribution towards inflammation, as dendrimers can colocalize with macrophages at wound sites, providing targeted delivery for the treatment of macrophage-mediated diseases or disorders.

The nanoglue is also useful for sealing of other types of injuries including sealing of dura, lung membrane, peritoneum, gastrointestinal, endothelium, and burns. The nanoglues have other applications. For example, the nanoglues can be used for sustained release of amoxicillin for intrauterine and genital tract infections.

### BRIEF DESCRIPTION OF THE DRAWINGS

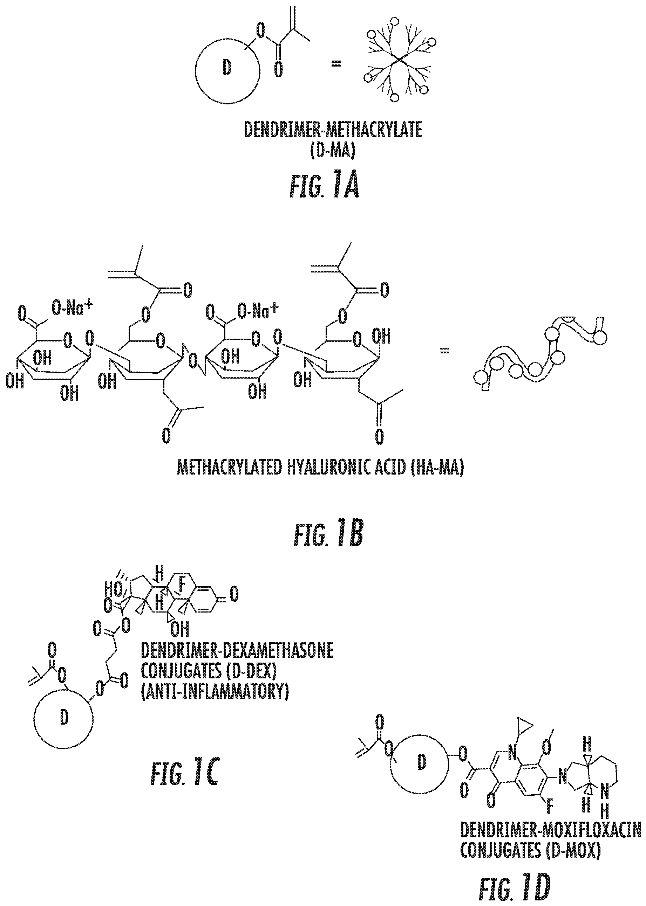

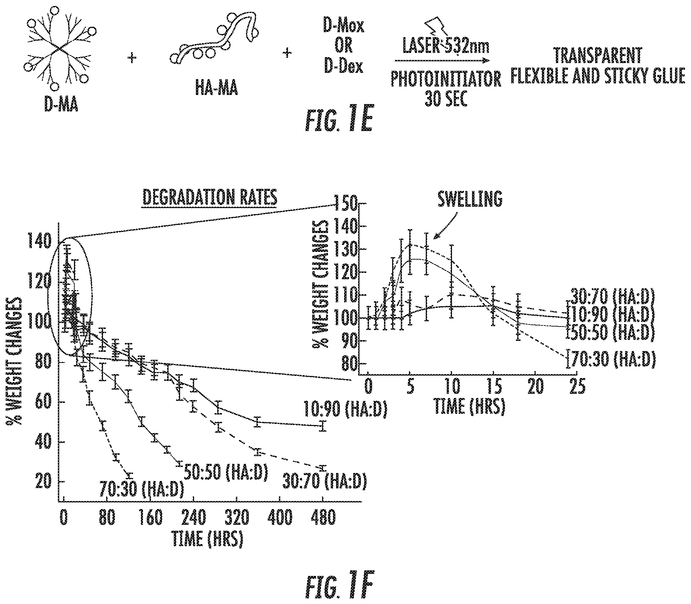

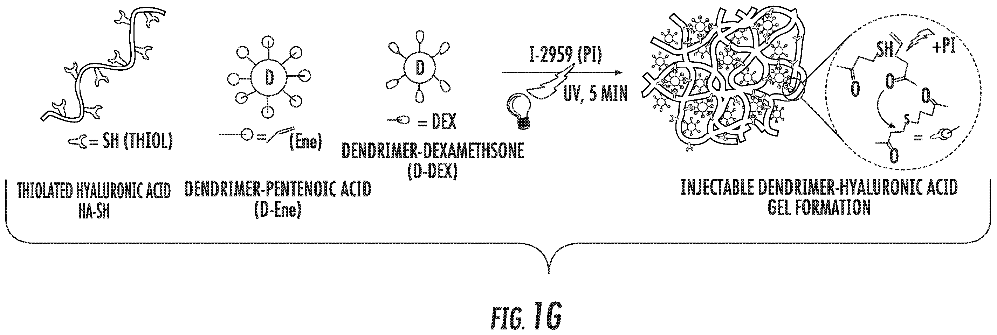

Figures 1A-1G are schematics of different components and the formation of dendrimer-hyaluronic acid hydrogel as transparent, flexible, and sticky glue. Figure 1A is a schematic of a dendrimer conjugated with one or more photo-cross-linkable groups of methacrylate, forming the D-MA conjugate. Figure 1B is a schematic of a segment of hyaluronic acid conjugated with one or more methacrylate, forming a HA-MA conjugate. Figure 1C is a schematic of D-MA further conjugated with an anti-inflammatory drug, (Steroid) dexamethasone, forming the MA-D-Dex conjugate. Figure 1D is a schematic of D-MA further conjugated with an anti-bacterial drug, moxifloxacin, forming the MA-D-Mox conjugate. Figure 1E is a schematic of the formation of drug-conjugated, photo-activated dendrimer-hyaluronic acid hydrogel. Figure 1F shows swelling and degradation rates for different ratios of dendrimer and hyaluronic acid components (HA:D) (10:90), (30:70), (50:50), (70:30) and (90: 10) of the nanoglue. Figure 1G is a schematic of formulation 2 of the nanoglue.

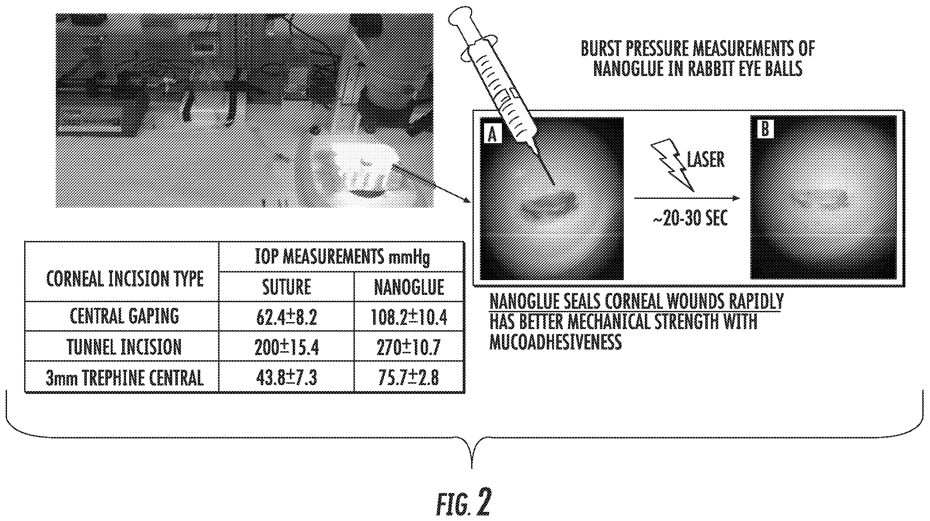

Figure 2 depicts ex-vivo evaluation of burst pressure and mechanical properties of nanoglue in fresh rabbit eyes: A 3mm linear incision was created in the central cornea resulting in gaping wound and the incision is sealed by applying the nanoglue solution and photo cured using laser for 30 seconds. The photopolymerization of nanoglue is indicated by transparent gel formation and the sealant withstands high intraocular pressure avoiding leakage of the fluid than compared to sutures. Inset Table: ex-vivo intraocular pressure (IOP) measurements in freshly enucleated rabbit eye balls: IOP measurements were measured using custom made saline infusion system equipped with digimanomanometer. The incisions sealed with nanoglue withstands high IOP avoiding leakage than compared to sutures suggests its strong adhesion to the cornea than compared to sutures.

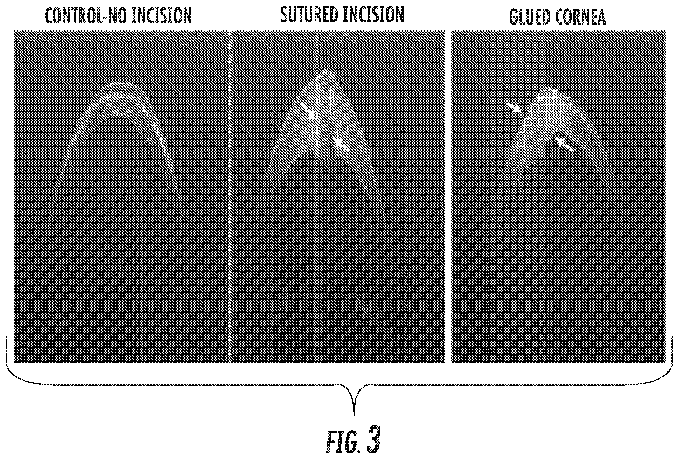

Figure 3 shows optical transmission tomography (OCT) imaging of rat laceration corneas: OCT is used to evaluate the efficacy of nanoglue in sealing the corneal laceration and wound healing. Left panel is OCT image of normal cornea with good corneal tissue architecture. The middle panel is corneal with laceration sutured with 10-0 suture resulting in fibrosis and corneal cleavage (yellow arrows) indicating improper wound healing on day 7. The right panel is the corneal laceration sealed with nanoglue (white arrows) with minimal fibrosis and better wound healing.

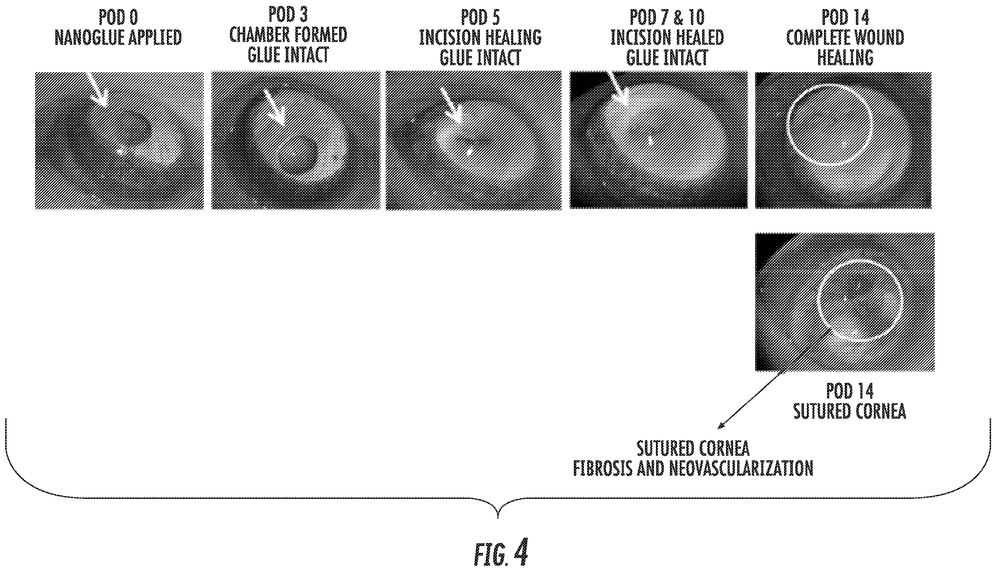

Figure 4 depicts clinical observation of efficacy of nanoglue in sealing and aiding wound healing of corneal incisions in rat corneal laceration model: A 3mm linear corneal laceration was created on rat cornea using a keratome knife and sealed using nanoglue formulation resulting in formation transparent barrier (white arrows). The animals were clinically assessed for wound healing until day 14 after nanoglue, all the rats with nanoglue resulted in enhanced corneal healing where as in sutured cornea resulted in corneal neovascularization and inflammation indicated with white arrows at the left corner of the panel.

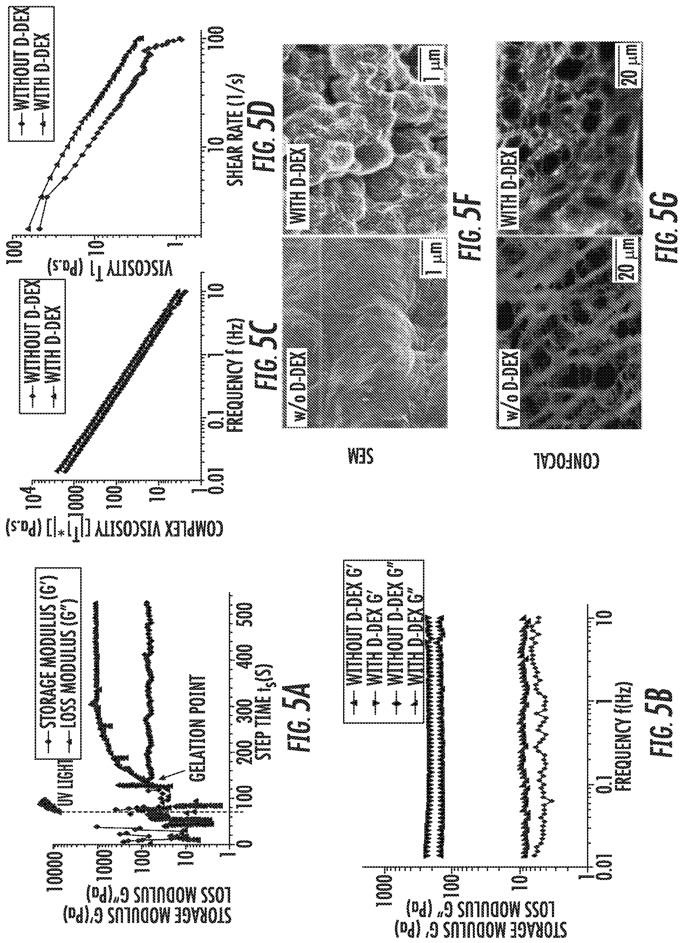

Figures 5A-G Characterization of injectable gels using rheology and SEM. 5A) Dynamic time sweep photo-rheology of injectable gel formation. The gelation point is approximated when storage modulus (G') overcomes the loss modulus (G") (arrow). Dashed line (---) illustrates when the UV light was turned on. 5B) Frequency sweep measurements of the injectable gel demonstrating their viscoelastic behavior (G'>>G"). 5C) viscosity vs frequency plot of injectable gel with and without D-Dex showing similar dynamic viscosity. 5D) Viscosity vas shear rate plots of injectable gels with and w/o D-Dex showing shear thinning properties. 5F) SEM images of dehydrated injectable gels with and without D-Dex showing the surface morphology. 5G) Confocal images of the FITC stained gel sections demonstrating the inner morphology with porous structure.

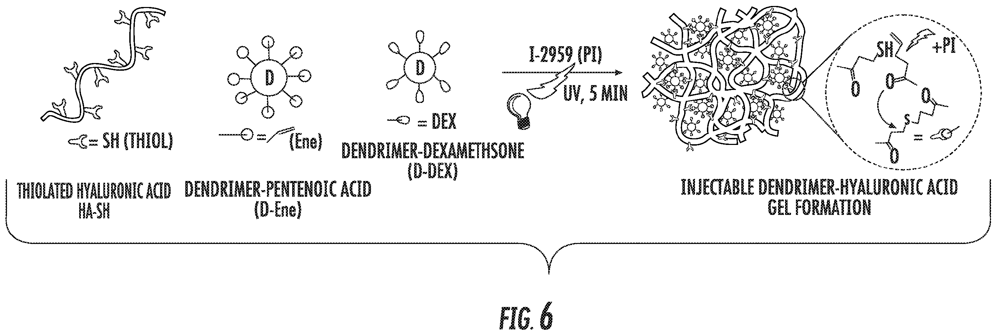

Figure 6 is a schematic of photo-activated, dendrimer-hyaluronic acid hydrogel, formed with thiolated hyaluronic acid (HA-SH), dendrimer-pentenoic acid conjugate (D-Ene), and dendrimer-dexamethasone conjugate (D-Dex) in the presence of Irgacure^{®} 2959 (I-2959). Here D-Dex is physically entrapped in the gel formulation.

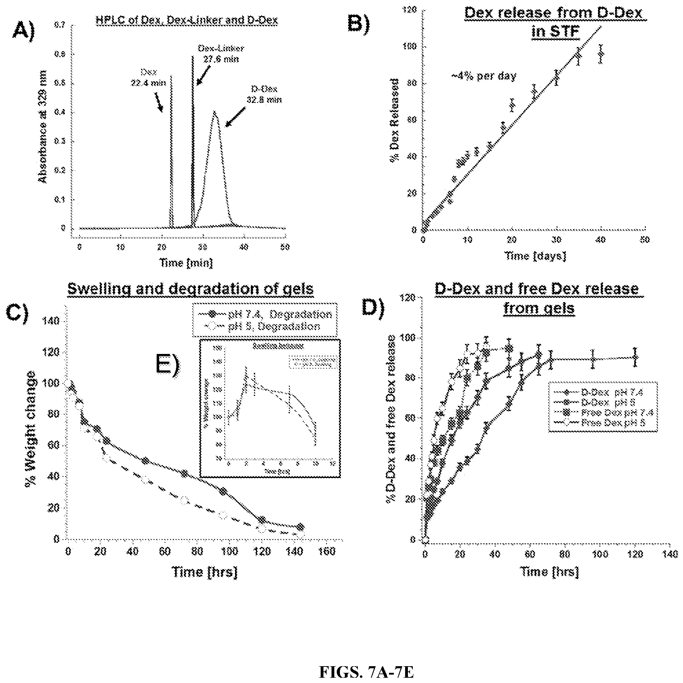

Figures 7A-7E depict the characterization of prepared conjugates. 7A) HPLC chromatograms of D-Dex (32.8 min), Dex (22.4 min) and Dex-Linker (27.6 min) monitored at 239 nm. 7B) Drug release profile from D-Dex conjugates in simulated tear fluid. 7C) Swelling and degradation profile of injectable gel in pH 7.4 and 5 respectively. 7D) D-Dex and Free Dex release profile from injectable gel formulations in pH 7.4 and 5 respectively. 7E) inset - weight change measurements depicting the swelling behavior of the gel.

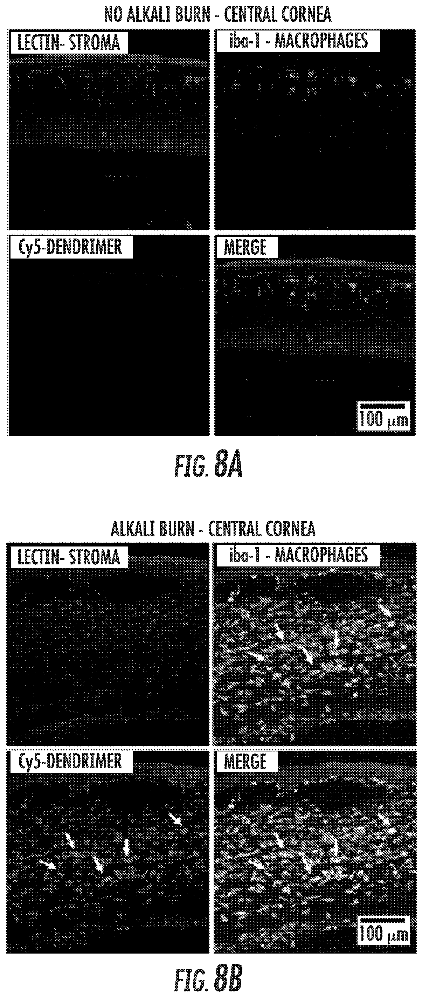

Figures 8A-B Biodistribution of subconjunctivally injected dendrimers. Fluorescently labelled dendrimers (D-Cy5) in gel formulations were injected subconjunctivally and the biodistribution was assessed 7 days after injection. Corneal stroma (Blue, Lectin), Macrophages (Green, Iba-1), Dendrimer (Red, Cy5). 8A) A central cross section of a normal cornea with regular tissue architecture; very few corneal Iba-1 stained cells (macrophages) are present; dendrimers are not co-localized in the macrophages. 8B) An alkali burnt central cornea infiltrated with Iba-1 positive cells (macrophages). Cy5 signals (dendrimer) are co-localized in the Iba-1 stained cells demonstrating dendrimer's intrinsic targeting capability towards inflammation. Scale bar 100 µm.

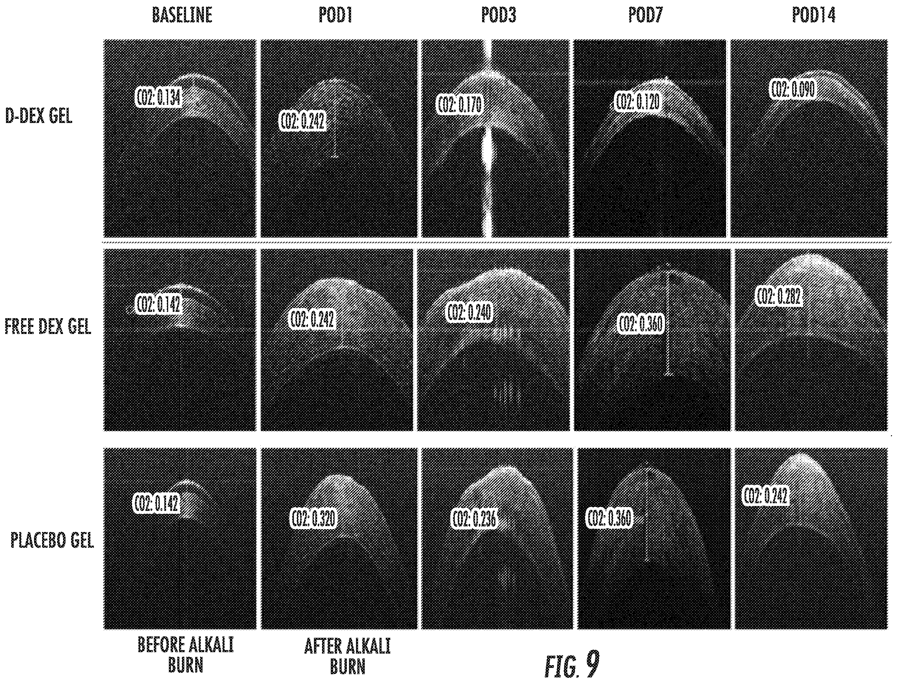

Figure 9 Anterior segment optical coherence tomography (OCT) imaging of the cornea for assessment of central corneal thickness (CCT). Top panel: OCT images of the central cornea of a D-Dex gel treated eye demonstrate near-normal corneal architecture at POD 7 and 14 when compared to its baseline. These images suggest that inflammation has subsided. Middle panel: OCT images of a central cornea treated with free-Dex gel demonstrate a thin irregular epithelial layer and stromal edema which suggest ongoing inflammation. Bottom panel: OCT images of a central cornea treated with placebo gel has similar characteristics as the free-Dex treated eye.

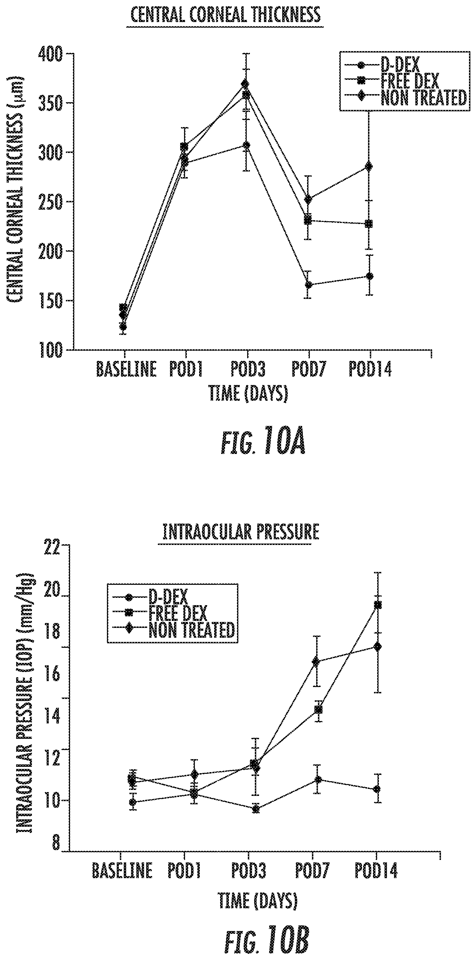

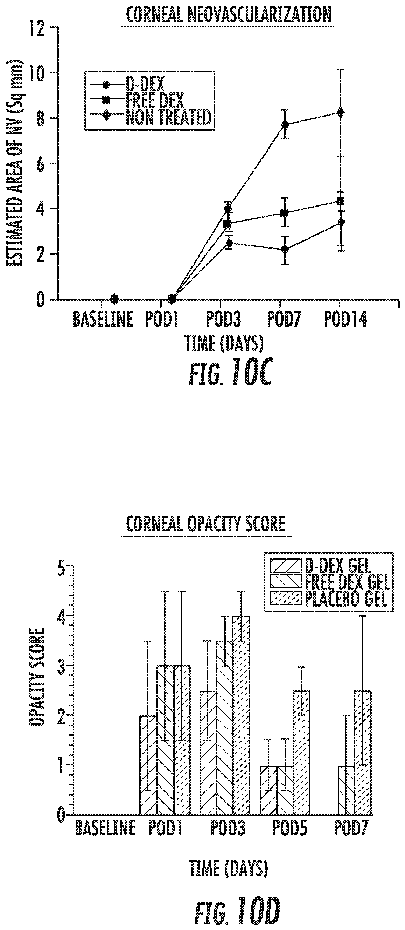

Figures 10A-10D are graphs showing ocular parameters of three groups of rats, each after alkali burn on the cornea, among (i) subconjunctival treatment with dendrimer-hyaluronic acid hydrogel with dendrimer-dexamethasone conjugate and a minimal amount of free dexamethasone (D-Dex), (ii) subconjunctival treatment with dendrimer-hyaluronic acid hydrogel with free dexamethasone (free Dex), and (iii) no treatment (untreated positive controls). Figure 10A is a line graph showing the central corneal thickness (CCT, µm) over time (postoperative days, PODs). Figure 10B is a line graph showing the intraocular pressure (IOP, mm/Hg) over time (PODs). Figure 10C is a line graph showing the estimated area of neovascularization (NV) (mm²) over time (PODs). Figure 10D is a bar graph showing the corneal opacity scores (median; range) over time (PODs).

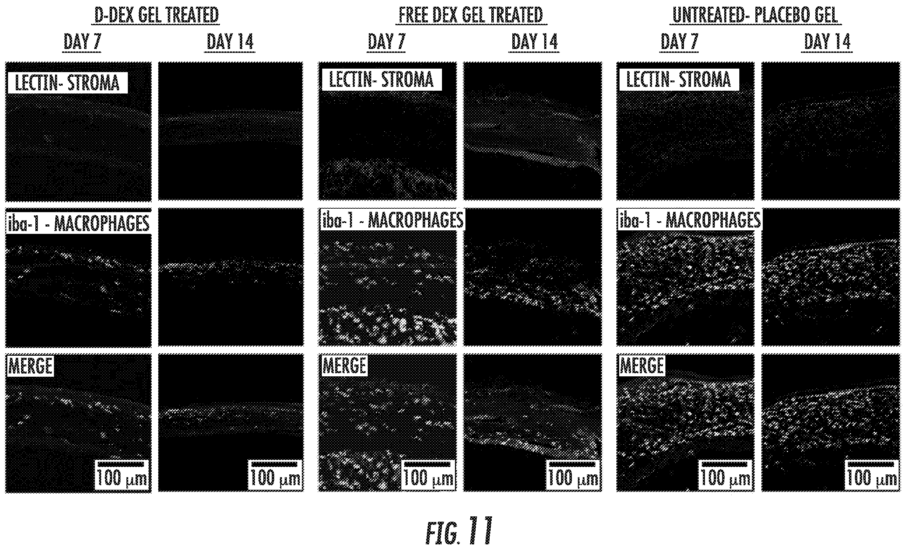

Figure 11 shows confocal microscope images of corneal cross sections. Left panel: a minimal amount of Iba-1 stained cellular infiltrate (macrophages) is observed at post-operative day 7 and 14. Middle pane and right panels: Unlike the D-Dex gel group, both free-Dex gel and placebo gel groups have a persistent IBA-1 stained cellular infiltrate (macrophages) at post-operative days 7 and 14. Scale bar 100 µm.

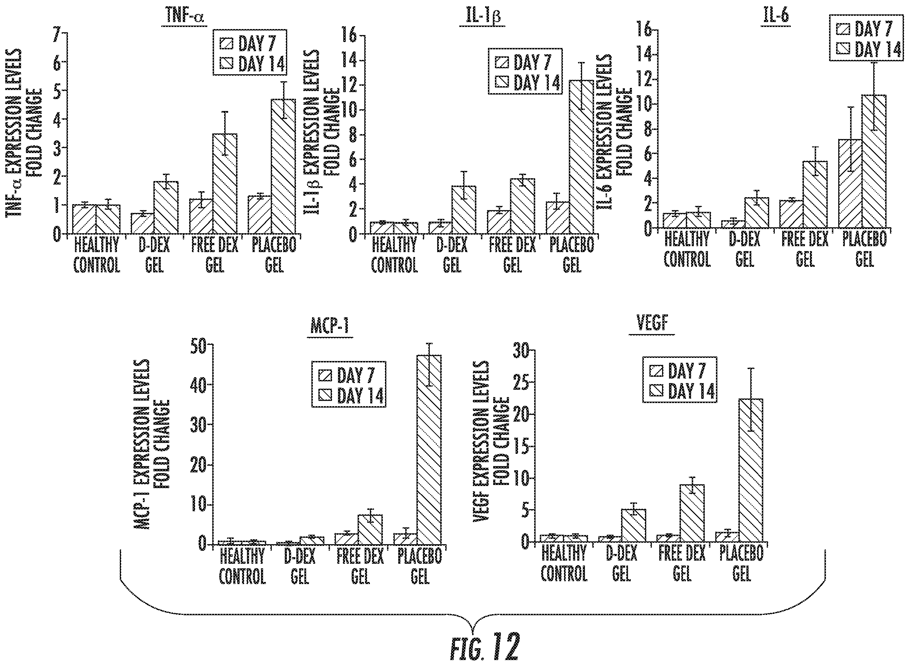

Figure 12 depicts bar graphs showing assessment of corneal inflammation after subconjunctival treatment by measuring the cytokine mRNA expression levels in corneal tissue using RT-PCR at POD 7 and 14. (TNF-α) - Tumor necrosis factor-α, (IL-1β) - Interleukin-1β, (IL-6) - Interleukin-6, (MCP-1) - Monocyte chemoattractant protein-1, (VEGF) - Vascular endothelial growth factor. The results are normalized to healthy controls and represented as mean ± SEM, n=10.

### DETAILED DESCRIPTION OF THE INVENTION

The invention is defined by the accompanying claims. Any subject matter falling outside the scope of the accompanying claims is provided as disclosure only.

### I. Definitions

The term "therapeutic agent" refers to an agent that can be administered to prevent or treat one or more symptoms of a disease or disorder. These may be a nucleic acid, a nucleic acid analog, a small molecule, a peptidomimetic, a protein, peptide, carbohydrate or sugar, lipid, or surfactant, or a combination thereof.

The term "diagnostic agent", as used herein, generally refers to an agent that can be administered to reveal, pinpoint, and define the localization of a pathological process.

The term "prophylactic agent", as used herein, generally refers to an agent that can be administered to prevent disease or to prevent certain conditions like pregnancy.

The phrase "pharmaceutically acceptable" refers to compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" refers to pharmaceutically acceptable materials, compositions or vehicles, such as a liquid or solid filler, diluent, solvent or encapsulating material involved in carrying or transporting any subject composition, from one organ, or portion of the body, to another organ, or portion of the body. Each carrier must be "acceptable" in the sense of being compatible with the other ingredients of a subject composition and not injurious to the patient.

The phrase "therapeutically effective amount" refers to an amount of the therapeutic agent that produces some desired effect at a reasonable benefit/risk ratio applicable to any medical treatment. The effective amount may vary depending on such factors as the disease or condition being treated, the particular targeted constructs being administered, the size of the subject, or the severity of the disease or condition. One of ordinary skill in the art may empirically determine the effective amount of a particular compound without necessitating undue experimentation. A prophylactic agent refers to an agent that may prevent a disorder, disease or condition. Examples include vaccines which prevent infection and birth control pills that prevent pregnancy.

The term "treating" refers to preventing or alleviating one or more symptoms of a disease, disorder or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain.

The term "biocompatible" as used herein, generally refers to materials that are, along with any metabolites or degradation products thereof, generally non-toxic to the recipient, and do not cause any significant adverse effects to the recipient. Generally speaking, biocompatible materials are materials which do not elicit a significant inflammatory or immune response when administered to a patient.

The term "biodegradable" as used herein, generally refers to a material that will degrade or erode under physiologic conditions to smaller units or chemical species that are capable of being metabolized, eliminated, or excreted by the subject. The degradation time is a function of composition and morphology. Degradation times can be from hours to years.

The term "seal", as used herein, means to substantially cover a rough surface, a cavity, or a gap in the substrate (e.g., a tissue) or coat on top of the tissue surface, and optionally to form a covalent or non-covalent bond at the contact surface. "Seal" may also refer to the barrier effect of preventing the migration or transport of certain gas, liquid, solute, macromolecules, or bacteria.

The term "dendrimer", as used herein, includes, but is not limited to, a molecular architecture with an interior core, interior layers (or "generations") of repeating units regularly attached to this initiator core, and an exterior surface of terminal groups attached to the outermost generation.

The term "bioadhesive polymer", as used herein, refers to a natural or synthetic polymer that can adhere to a biological substrate. The adhesion of polymers to tissues may be achieved by (i) physical or mechanical bonds, (ii) primary or covalent chemical bonds, and/or (iii) secondary chemical bonds (i.e., ionic).

The term "crosslink", as used herein, means the formation of covalent linkages between a precursor molecule containing nucleophilic groups and a precursor molecules containing electrophilic group resulting in an increase in the molecular weight of the material. "Crosslink" may also refer to the formation of non-covalent linkages, such as ionic bonds, or combinations of covalent and non-covalent bonds.

The term "photocrosslink", as used herein, means to cause vinyl or other unsaturated bonds to break and form cross-links by the application of radiant energy.

The term "external stimulus", as used herein, evokes a specific functional reaction, which is not intrinsic, such as a physical, chemical, biological, mechanical, and irradiation stimuli.

"Polymeric network", as used herein, refers to the product of a process in which substantially all of the monomers, oligomers, or polymers are bound by intermolecular covalent linkages through their available functional groups to form a macromolecule.

"Physiological", as used herein, refers to conditions found in living vertebrates. In particular, physiological conditions refer to the conditions in the human body such as temperature, pH, aqueous medium, etc. "Physiological temperatures", as used herein, refers to a temperature range of between 35°C to 42°C, preferably around 37°C.

"Crosslink density", as used herein, refers to the average molecular weight between two crosslinks (M_{c}) of the respective molecules.

"Swelling", as used herein, refers to the increase in volume and mass due to the uptake of water by the biomaterial. The terms" water-uptake" and "swelling" are used synonymously.

"Gel point" or "gelation" as used herein refers to the point where the viscous modulus and complex modulus cross each other and viscosity increases. Thus the gel point is the stage at which a liquid begins to take on the semisolid characteristics of a gel.

*"In situ* formation" as generally used herein refers to the ability of mixtures of precursor molecules which are substantially not crosslinked prior to and at the time of injection, but form covalent linkages, non-covalent linkages, or a combination, with each other at a physiological condition or upon trigger by external stimuli at the site of injection in the body.

"Equilibrium state", as used herein, refers to the state in which a hydrogel undergoes no mass increase or loss when stored under constant conditions in water.

"Functionalize", as used herein, means to modify in a manner that results in the attachment of a functional group or moiety. For example, a molecule may be functionalized by the introduction of a molecule which makes the molecule a strong nucleophile or strong electrophile. For example, a molecule, such as hyaluronic acid, may be functionalized to become a thiol, amine, acrylate, or quinone.

As used herein, the term "active agent" or "biologically active agent" are used interchangeably herein to refer to a chemical or biological compound that induces a desired pharmacological and/or physiological effect, which may be prophylactic, therapeutic or diagnostic. The terms also encompass pharmaceutically acceptable, pharmacologically active derivatives of active agents, including, but not limited to, salts, esters, amides, prodrugs, active metabolites, and analogs.

### II. Nanoglues

The nanoglue adheres to moist tissues, permits controlled placement, and allows for rapid curing to seal the wound. The nanoglue may be used for the delivery of therapeutic, prophylactic and/or diagnostic agents and thereby be useful to treat underlying disorders and accelerate wound healing.

### A. Hydrogel sealant or its precursors

Two main components are included: bioadhesive polymers that provide strength and flexibility and are biocompatible to integrate with wounded tissues and accelerate wound healing, and dendrimers that anchor with bioadhesive polymers to provide a high density of crosslinking points and thereby a desired mechanical strength.

In a preferred embodiment, the precursor components are modified with vinyl or other unsaturated groups and nucleophilic groups, independently, to allow for photo-crosslinking and formation of hydrogel *in situ.*

### i. Dendrimers

In preferred embodiments, the dendrimers and non-toxic and have numerous surface groups enabling modification with multiple photo-crosslinkable groups for high crosslinking densities at low concentrations and potential conjugation with active agents.

Dendrimers suitable for use include, but are not limited to, polyamidoamine (PAMAM), polypropylamine (POPAM), polyethylenimine, polylysine, polyester, iptycene, aliphatic poly(ether), and/or aromatic polyether dendrimers. Each dendrimer of the dendrimer complex may be same or of similar or different chemical nature than the other dendrimers (e.g., the first dendrimer may include a PAMAM dendrimer, while the second dendrimer may be a POPAM dendrimer). The first or second dendrimer may further include an additional agent such as a multiarm PEG polymer including a polyethylene glycol having at least two branches bearing sulfhydryl or thiopyridine terminal groups. Other PEG polymers bearing other terminal groups such as succinimidyl or maleimide terminations can be used. The PEG polymers in the molecular weight 10 kDa to 80 kDa can be used. Complexes can be formed of one or more dendrimers.

Examples of dendrimers include, but are not limited to, poly(amidoamine) (PAMAM), polyester, polylysine, and polypropylenimine (PPI). The PAMAM dendrimers may contain different cores, with amidoamine building blocks, and can have carboxylic, amine and hydroxyl terminations of any generation including, but not limited to, generation 1 PAMAM dendrimers, generation 2 PAMAM dendrimers, generation 3 PAMAM dendrimers, generation 4 PAMAM dendrimers, generation 5 PAMAM dendrimers, generation 6 PAMAM dendrimers, generation 7 PAMAM dendrimers, generation 8 PAMAM dendrimers, generation 9 PAMAM dendrimers, or generation 10 PAMAM dendrimers. In the preferred embodiment, the dendrimers are soluble in the formulation and are generation ("G") 4, 5 or 6 dendrimers.

As used herein, the term "PAMAM dendrimer" means poly(amidoamine) dendrimer, which may contain different cores, with amidoamine building blocks. The method for making them is known to those of skill in the art and generally involves a two-step iterative reaction sequence that produces concentric shells (generations) of dendritic β-alanine units around a central initiator core. This PAMAM core-shell architecture grows linearly in diameter as a function of added shells (generations) and the surface groups amplify exponentially at each generation according to dendritic-branching mathematics. They are available in generations G0 - 10 with 5 different core types and 10 functional surface groups. The dendrimer-branched polymer may consist of polyamidoamine (PAMAM), polyglycerol, polyester, polyether, polylysine, or polyethylene glycol (PEG), polypeptide dendrimers. The dendrimers may have hydroxyl groups attached to their functional surface groups.

The dendrimers may be in nanoparticle form and are described in detail in international patent publication Nos. WO2009/046446, PCT/US2015/028386, PCT/US2015/045112, PCT/US2015/045104, and U.S. Patent No. 8,889,101.

### ii. Bioadhesive polymers

Bioadhesive polymers include natural or synthetic polymers that adhere to biological tissues. As used herein, bioadhesive polymers adhere through covalent bonding, non-covalent interactions (e.g., hydrogen bond), and/or physical entanglement with tissue substrates.

In some specific embodiments, bioadhesive polymers refer to mucoadhesive polymers for adhesion in tissues in the respiratory, nasal, cervicovaginal, gastrointestinal, rectal, visual and auditory systems.

### 1. Hyaluronic acid (HA)

HA is a naturally occurring, immunoneutral glycosaminoglycan of the extracellular matrix that plays an important role in development, wound healing, and inflammation. Its viscoelastic properties and long ocular surface residence time have rendered it suited for use in several tissue repairing practices, including ophthalmic use to protect the corneal endothelium. HA is also identified to be a ligand for CD44 (Zhu SN, et al., Br J Ophthalmol., 81(1):80-4 (1997)), a transmembrane cell surface adhesion molecule.

In a preferred embodiment, HA crosslinked by dendrimer molecules forms the hydrogel sealant for tissue repair. HA has a wide range of molecular weights; and in some embodiments, HA of 15-20kDa is selected. To crosslink, HA is first modified chemically at one or more of the three functional groups: the glucuronic acid carboxylic acid, the primary and secondary hydroxyl groups, and the N -acetyl group (following deamidation). Most prominently, carboxylates have been modified by carbodiimide-mediated reactions, esterification, and amidation; hydroxyls have been modified by etherification, divinylsulfone crosslinking, esterification, and bis-epoxide crosslinking. Detailed reviews on chemical modification of HA can be seen in Kuo JW, Prestwich GD, in Materials of Biological Origin - Materials Analysis and Implant Uses, Comprehensive Biomaterials, Elsevier (2010).

### 2. Other bioadhesive polymers

Bioadhesive polymers that can be incorporated include synthetic and natural polymers. Preferred bioadhesive polymers have exposed carboxylic groups. These include natural polymers such as alginates and celluloses and synthetically modified celluloses including alkyl celluloses, hydroxyalkyl celluloses, cellulose ethers, cellulose esters, and nitrocelluloses, such as carboxymethylcellulose, hydroxymethylcellulose and methylcellulose. Synthetic polymers that can be used include polyesters such as poly(hydroxyesters) like polylactide-co-glycolide, polylactide, and polyglycolide, polyorthoesters, polyanhydrides, polyhydroxyalkanoates such as poly3hydroxybutyrate, poly4hydroxybutyrate and copolymers thereof, and non-biodegradable polymers such as acrylates and methacrylates, copolymers and derivatives thereof, poly(vinyl alcohols), polyamides, and polycarbonates. Blends and copolymers made include polyalkylenes, polyalkylene glycols, polyalkylene oxides, polyalkylene terephthalates, polyvinyl ethers, polyvinyl esters, polyvinyl halides, polyvinylpyrrolidone, polyglycolides, polysiloxanes, polyurethanes and copolymers thereof. All polymers are commercially available.

### iii. Modifications to permit stimuli-responsive gelation

Dendrimers and bioadhesive polymers are modified with crosslinking moieties to enable stimuli-responsive gelation. The dendrimers and the bioadhesive polymers are chemically modified to contain photo-crosslinkable groups (e.g., thiol groups and vinyl groups; thiol groups and maleimide groups; amine groups and aldehyde groups; amine groups and carboxylate groups; methacrylate groups; and acrylate groups).

In some embodiments, one or more methacrylate or acrylate group is modified onto both dendrimers and bioadhesive polymers, or onto one species while the other species is modified with a thiol group. These modifications permit photo-crosslink to cure precursor materials and form the nanoglue.

In another embodiment, dendrimers and bioadhesive polymers are modified to permit the clickable, thiol-ene or thiol-yne reactions, wherein one species is functionalized to terminate with a thiol group whereas the other species is modified with an alkene or alkyne functional group. For example, an alkene can be norbornene. Sometimes, these thiol-ene or thiol-yne reactions are facilitated by photo irradiation.

In yet other embodiments, crosslinking is achieved with thiol-maleimide, amine-aldehyde or amine carboxylate reactions, and the dendrimers and bioadhesive polymers are modified with relevant groups.

In photo-crosslink reactions, photo-initiators can be included. Photoinitiators are compounds that, under absorption of light, undergo a photoreaction, producing reactive species that are capable of initiating the crosslink of the unsaturated constituents. Exemplary photoinitiators include IRGACURE^{®} compounds.

In some embodiments, the photo-crosslinked hydrogel is a transparent, flexible gel that is suited for ocular treatment.

### B. Therapeutic, Prophylactic and Diagnostic Agents

The nanoglue may include one or more therapeutic, prophylactic, or diagnostic agents that are encapsulated, conjugated to the components of the hydrogel, or encapsulated in/conjugated to sustained release nanoparticle/microparticle formulations that are dispersed in the hydrogel precursor(s). In some embodiments, the agents can be modified with a succinate group via a reaction with succinic anhydride, which are later conjugated to dendrimers and/or bioadhesive polymers at their hydroxyl groups to permit ester linkage and hydrolysis-mediated, sustained release of the agents in tissue.

Representative therapeutic agents include, but are not limited to, anti-inflammatory drugs, including immunosuppressant agents and anti-allergenic agents, and antiinfectious agents. Some examples of anti-inflammatory drugs include steroids like triamcinolone acetonide, fluocinolone acetonide, prednisolone, dexamethasone, loteprendol, fluorometholone. Immune modulating drugs such as: cyclosporine, tacrolimus and rapamycin. Non steroidal anti inflammatory drug include ketorolac, nepafenac, and diclofenac. Antiinfectious agents include antiviral agents, antibacterial agents, antiparasitic agents, and anti-fungal agents. Exemplary antibiotics include moxifloxacin, ciprofloxacin, erythromycin, levofloxacin, cefazolin, vancomycin, tigecycline, gentamycin, tobramycin, ceftazidime, ofloxacin, gatifloxacin; antifungals: amphotericin, voriconazole, natamycin.

Active agents can include anti-glaucoma agents that lower intraocular pressure (IOP), anti-angiogenesis agents, growth factors, and combinations thereof. Examples of anti-glaucoma agents include prostaglandin analogs such as travoprost and latanoprost, prostamides such as bimatoprost; beta-adrenergic receptor antagonists such as timolol, betaxolol, levobetaxolol, and carteolol, alpha-2 adrenergic receptor agonists such as brimonidine and apraclonidine, carbonic anhydrase inhibitors such as brinzolamide, acetazolamine, and dorzolamide, miotics (i.e., parasympathomimetics) such as pilocarpine and ecothiopate), seretonergics, muscarinics, and dopaminergic agonists.

Representative anti-angiogenesis agents include, but are not limited to, antibodies to vascular endothelial growth factor (VEGF) such as bevacizumab (AVASTIN^{®}) and rhuFAb V2 (ranibizumab, LUCENTIS^{®}), and other anti-VEGF compounds including aflibercept (EYLEA^{®}); MACUGEN^{®} (pegaptanim sodium, anti-VEGF aptamer or EYE001) (Eyetech Pharmaceuticals); pigment epithelium derived factor(s) (PEDF); COX-2 inhibitors such as celecoxib (CELEBREX^{®}) and rofecoxib (VIOXX^{®}); interferon alpha; interleukin-12 (IL-12); thalidomide (THALOMID^{®}) and derivatives thereof such as lenalidomide (REVLIMID^{®}); squalamine; endostatin; angiostatin; ribozyme inhibitors such as ANGIOZYME^{®} (Sirna Therapeutics); multifunctional antiangiogenic agents such as NEOVASTAT^{®} (AE-941) (Aeterna Laboratories, Quebec City, Canada); receptor tyrosine kinase (RTK) inhibitors such as sunitinib (SUTENT^{®}); tyrosine kinase inhibitors such as sorafenib (Nexavar^{®}) and erlotinib (Tarceva^{®}); antibodies to the epidermal grown factor receptor such as panitumumab (VECTIBIX^{®}) and cetuximab (ERBITUX^{®}), as well as other anti-angiogenesis agents known in the art.

In some cases, the active agent is a diagnostic agent imaging or otherwise assessing the eye. Examples of diagnostic agents include paramagnetic molecules, fluorescent compounds, magnetic molecules, and radionuclides, x-ray imaging agents, and contrast media.

The active agents may be present in their neutral form, or in the form of a pharmaceutically acceptable salt. In some cases, it may be desirable to prepare a formulation containing a salt of an active agent due to one or more of the salt's advantageous physical properties, such as enhanced stability or a desirable solubility or dissolution profile.

Generally, pharmaceutically acceptable salts can be prepared by reaction of the free acid or base forms of an active agent with a stoichiometric amount of the appropriate base or acid in water or in an organic solvent, or in a mixture of the two; generally, non-aqueous media like ether, ethyl acetate, ethanol, isopropanol, or acetonitrile are preferred. Pharmaceutically acceptable salts include salts of an active agent derived from inorganic acids, organic acids, alkali metal salts, and alkaline earth metal salts as well as salts formed by reaction of the drug with a suitable organic ligand (e.g., quaternary ammonium salts). Lists of suitable salts are found, for example, in Remington's Pharmaceutical Sciences, 20th ed., Lippincott Williams & Wilkins, Baltimore, MD, 2000, p. 704. Examples of ophthalmic drugs sometimes administered in the form of a pharmaceutically acceptable salt include timolol maleate, brimonidine tartrate, and sodium diclofenac.

In certain embodiments, the nanoglue contains one or more local anesthetics. Representative local anesthetics include tetracaine, lidocaine, amethocaine, proparacaine, lignocaine, and bupivacaine. In some cases, one or more additional agents, such as a hyaluronidase enzyme, is also added to the nanoglue to accelerate and improves dispersal of the local anesthetic.

### C. Formulations

The nanoglue can be administered as a liquid, or in a solution with one or more pharmaceutically acceptable excipients in one or more pharmaceutically acceptable carrier. Representative excipients include solvents, diluents, pH modifying agents, preservatives, antioxidants, suspending agents, wetting agents, viscosity modifiers, tonicity agents, stabilizing agents, and combinations thereof. Suitable pharmaceutically acceptable excipients are preferably selected from materials which are generally recognized as safe (GRAS), and may be administered to an individual without causing undesirable biological side effects or unwanted interactions.

Pharmaceutical compositions may be for administration to the eye compartment to be treated (e.g., vitreous, subretinal space, subchoroidal space, the episclera, the conjunctiva, the subconjunctiva, the sclera, the anterior chamber, and the cornea and compartments therein, e.g., subepithelial, intrastromal, endothelial), or corneal surface, and can be formulated in unit dosage forms appropriate for each route of administration.

Pharmaceutical compositions for other uses include nanoglues formulated for sustained release of antibiotics for intrauterine and genital tract infections. The nanoglue can also be formulated for sealing of other types of injuries including sealing of dura, lung membrane, peritoneum, gastrointestinal, endothelium, and burns

A benefit of the nanoglue is the targeted biodistribution towards inflammation, as dendrimers can colocalize with macrophages at wound sites, providing targeted delivery for the treatment of macrophage-mediated diseases or disorders.

### D. Kits

In some embodiments, the compositions are provided in a kit. Formulations are prepared using a pharmaceutically acceptable "carrier" composed of materials that are considered safe and effective and may be administered to an individual without causing undesirable biological side effects or unwanted interactions. Typically the nanoglue will be in a single dose unit, or in a kit with a first containing with liquid to rehydrate the dry components in a second component. These may include components for administration, such as a dropper or another applicator device such as a dual barrel syringe, for example.

### III. Methods of Making the Composition

The method for making dendrimers is known to those of skill in the art and generally involves a two-step iterative reaction sequence that produces concentric shells (generations) of dendritic β-alanine units around a central initiator core. This PAMAM core-shell architecture grows linearly in diameter as a function of added shells (generations). Meanwhile, the surface groups amplify exponentially at each generation according to dendritic-branching mathematics. They are available in generations G0 - 10 with 5 different core types and 10 functional surface groups. The dendrimer-branched polymer may consist of polyamidoamine (PAMAM), polyester, polyether, polylysine, or polyethylene glycol (PEG), polypeptide dendrimers. Alternatively, dendrimers with various termination groups can be purchased from vendors such as Dendritech.

Modifications to dendrimers and/or bioadhesive polymers to allow for stimuli-triggered gelation are known with various functional groups. Thiolation of hyaluronic acid is described in the literature. Kafedjiiski K, et al., Int J Pharm, 343(1-2):48-58 (2007) reported the synthesis of thiolated hyaluronic acid by conjugating L-cysteine ethyl ester to the carboxyl (-COOH) groups of the hyaluronic acid using EDC-NHS coupling reaction in aqueous solvent. The resultant product was subjected to oxidation to form disulfide (-S-S-) bridges, which permits HA to form gels for drug delivery. As an alternative, free thiols on hyaluronic acid can be generated to allow for thio-ene click reactions. In this approach, since a reaction in the aqueous medium may result in disulfide formation, an inert condition for this modification reaction is preferred, which provides better control on the degree of substitution and avoids *in-situ* disulfide formation. Detailed procedures for this modification are explained in the Examples section.

Active agents can also be modified to permit covalent attachment and controlled release. Alternatively, active agents can be mixed with one or both of the precursor components, and are dispersed in the formed hydrogel.

In photocrosslinking, the intensity of the irradiation, the exposure time, the amount of photoinitiator, and/or the concentration of the hydrogel precursor components can be adjusted to control how fast the precursor solutions are cured and to tailor the mechanical properties of the formed hydrogel. In preferred embodiments, a low intensity of UV irradiation allows for the gelation of precursor components within one minute, preferably with 30 seconds, which does not pose damages to the retinal cells.

### IV. Methods of Using the Composition

The nanoglue can be used as a sealant for tissue wounds or surgical incisions, for injectable delivery of small molecules, micro/nanoparticles, DNA/siRNAs, etc. for treatment of diseases or disorders, and as instillation formulations for sustained and enhanced permeable delivery of agents to mucosal surfaces. In one embodiment, the hydrogel precursor solution is for use in a method where it is administered to tissue sites, and the stimuli-triggered gelation takes place *in situ.* Alternatively, a formed, injectable hydrogel may be injected into tissue sites to form a depo. Different methods of applying the nanoglue is based on the kinetics of the chemical groups in the nanoglue, the sites of tissues, and the need of the practitioners.

### A. Ocular Applications

### Corneal wound

The cornea of the eye serves an important role in refracting and focusing light rays necessary for clear vision. The cornea possesses the unique characteristics of an orderly arrangement of stromal collagen fibrils and a lack of blood vessels that result in transparency.

Corneal wounds arise from surgical procedures (e.g., transplants, incisions for cataract removal and intraocular lens implantation, laser-assisted *in situ* keratomileusis), infections (e.g., ulcers), and traumatic injury (e.g., lacerations, perforations).

In a preferred embodiment, the hydrogel formed from hyaluronic acid and dendrimers that crosslink upon UV irradiation is applied as a sealant for corneal wounds, where the precursors are applied to the corneal wound and are photo-cured *in situ* in a tailored manner.

### B. Other wounds or surgical incisions

The nanoglue may be used in sealing intra-amniotic ruptures or surgical incisions, sealing air leaks in both open and minimally invasive thoracic surgery, sealing vasculature at vascular reconstruction sites, sealing and reducing anastomotic leakage in gastrointestinal surgical procedures, and sealing wounds in cardiovascular surgery.

Compared with the currently available adhesives or sealants, i.e., fibrin, cyanoacrylates, gelatin/thrombin products, PEG polymer, and albumin/glutaraldehyde products, the nanoglue is advantageous with respect to biocompatibility, low toxicity, transparency, support for versatile modifications and drug conjugations, and tunability for gelling kinetics.

### C. Disorders to be treated

In addition to sealing tissues, the nanoglue can be used for local delivery of active agents. In the treatment of ocular diseases or disorders, topical antibiotics and steroids reduce the risk of infection and corneal scarring. However, the drugs are often cleared through tear secretions, which often leads to repeated dosing, elevated intraocular pressure, corneal irritation and toxicity, and potentially discoloration of cornea, (Gibson JM, et al., US Ophthalmic Review, 8.1:2-7 (2015); Mahajan SH, Carbohydr Polym, May 20; 122:243-7 (2015)).

The nanoglue forms transparent flexible injectable gels that can be injected intravitreally for sustained release of drugs to treat a variety of posterior segment diseases, e.g., diabetic retinopathy, choroidal neovascularization (CNV), and age related macular degeneration (AMD). Further examples of eye disorders that may be treated include amoebic keratitis, fungal keratitis, bacterial keratitis, viral keratitis, onchorcercal keratitis, bacterial keratoconjunctivitis, viral keratoconjunctivitis, corneal dystrophic diseases, Fuchs' endothelial dystrophy, Sjogren's syndrome, Stevens-Johnson syndrome, autoimmune dry eye diseases, environmental dry eye diseases, corneal neovascularization diseases, post-corneal transplant rejection prophylaxis and treatment, autoimmune uveitis, infectious uveitis, anterior uveitis, posterior uveitis (including toxoplasmosis), pan-uveitis, an inflammatory disease of the vitreous or retina, endophthalmitis prophylaxis and treatment, macular edema, macular degeneration, age related macular degeneration, proliferative and non-proliferative diabetic retinopathy, hypertensive retinopathy, autoimmune disease of the retina, primary and metastatic intraocular melanoma, other intraocular metastatic tumors, open angle glaucoma, closed angle glaucoma, pigmentary glaucoma and combinations thereof.

The dendrimer-bioadhesive polymer nanoglue may provide sustained release of a therapeutic agent over a period of time. For example, after administration, the therapeutic agent can be released for at least 6 hours, at least 12 hours, at least 1 day, at least 2 days, at least 3 days, at least one week, at least 2 weeks, at least 3 weeks, at least 4 weeks, at least 5 weeks, at least 6 weeks, at least 7 weeks, at least 2 months, at least 3 months, at least 6 months, at least 9 months, at least 1 year or more.

Administration of the hydrogel or hydrogel precursors followed by photo irradiation-mediated curing causes less inflammation or increase in IOP over time than if other drug delivery systems are administered. A reduction relative to inflammation or increase in I|OP using sutures of inflammation or IOP can be observed after about 3 days post-administration, until 14 days post-administration or more. Inflammation or IOP is reduced over time, even with administration of conventional or previously available drug delivery systems. However, the reduction or avoidance of inflammation or IOP when using methods and compositions disclosed herein is persistent and more significant. The reduction or avoidance of inflammation or IOP can continue for at least about 3 days, at least about 14 days, at least about 20 days, or at least about 30 days post-administration.

The present invention will be further understood by reference to the following nonlimiting examples.

### Example 1. Preparation of photocrosslinked, dendrimer-hyaluronic acid hydrogel with covalently attached dendrimer-drug conjugates.

### Materials and methods

### Modification of dendrimer with methacrylate (D-MA)(Formulation 1)

The surface of PAMAM G2 and G4 hydroxyl dendrimers are modified with methacrylate groups thereby enabling photo crosslinking (*Component 1*). Photo crosslinkable dendrimers (G2, G3, G4 and G6) are synthesized by adopting and optimizing previously established lab procedures. Briefly, methacrylic acid was covalently conjugated to the surface groups of the dendrimer using PyBOP/DIEA coupling reaction and the resultant product was purified and dialyzed using water. The formation of the product (D-MA) was confirmed using ¹H NMR and HPLC (Figure 1A).

### Synthesis of photo-crosslinkable, dendrimer-drug conjugate (Component 2)

Synthesized dendrimer dexamethasone conjugates with photocrosslinkable groups (MA-D-Dex) (G2-G6) were made that can enhance wound healing and prevent corneal scarring and neovascularization. The drugs are not restricted to triamcinolone acetonide, budesonide, fluocinolone acetonide, cyclosporine, rapamycin and etc (prednisolone, dexamethasone, loteprendol, fluorometholone, ketorolac, nepafenac, diclofenac, cyclosporine, tacrolimus) (Figure 1C).

### Synthesis of a photocrosslinkable dendrimer anti-biotic component (Component 3)

Dendrimer moxifloxacin conjugates were synthesized with photocrosslinkable groups (MAD-Mox) that can provide antibacterial barrier and avoids any chance of infection which often results in corneal inflammation, endothaplmities and corneal opacity. The antibiotics are not restricted to antibacterial-ciprofloxacin, erythromycin, levofloxacin, cefazolin, antiviral-vancomycin, gentamycin, tobramycin, ceftazidime, ofloxacin, gatifloxacin, amphotericin, voriconazole, natamycin, and antifungal agents (Figures 1D, 1E). We have also synthesized dendrimer-cefazolin (D-Cef) and dendrimer-tobramycin (D-Tob)

### Modification of hyaluronic acid with methacrylate (HA-MA)(Component 4)

Hyaluronic acid- methacrylate (HA-MA) was synthesized using 2 step procedure. The first step is conversion of commercially available sodium hyluronate (Na-HA) to TBA salt (HA-TBA) and the 2^{nd} step involves covalently modifying the hydroxyl groups on the hyaluronic acid with methacrylic acid using PyBOP/DIEA coupling reaction (Figure 1B).

Photocrosslinkable hyaluronic acid was synthesized with high degree of methacrylation that enables better crosslinking with the dendrimer and provides flexibility without compromising the mechanical properties of the nanoglue. The polymers are not restricted to collagen, chondroitin sulfate, pullulan, chitosan, cyclodextrin, poly ethylene glycol (linear and star PEG), poly-L-Lysine, PLGA, PGA, Poly capro-lactone etc. (Figure 1C)

*Choosing appropriate ratios (D:HA):* We used different ratios of dendrimer and hyaluronic acid components (HA:D) (10:90), (30:70), (50:50), (70:30) and (90: 10) to optimize the swelling, strength and stability of the glue. The stability and swelling studies were performed in simulated tear fluid (pH 7.0). We found that if we increase the HA content swelling properties increase and the stability reduces thereby the composition degrades at a faster rate. The nanoglue formulation with polymer ratios of (10:90) and (30:70) did not show significant swelling and were stable for a period of 20 days. From the stability studies, the appropriate composition was (30:70) as the nanoglue is flexible and stable (Figure 1F).

### Photocrosslink formation of nanoglue

D-MA, HA-SH, and D(-MA)-Dex or D(-MA)-Mox were premixed. An argon green laser (325 nm) was applied for 20-30 seconds.

### Results

Figures 1A-1F show the procedures of forming nanoglue. The formed hydrogel was transparent, flexible, and sticky.

### Example 2. Optical and mechanical properties of nanoglue compared to sutures in ex vivo wounded rabbit eyeballs.

### Materials and methods

Different types of corneal incisions were created on freshly enucleated mature rabbit eyeballs. We used different wound architecture and measured the burst pressure using custom designed manometer system with saline infusion. The nanoglue formulation with premixed formulations were applied on corneal incisions with different wound architecture and a argon green laser (325 nm) (for methacrylate system, formulation 1) or cobalt blue light (for thiol-ene click chemistry, formulation 2) was applied for 20-30 seconds resulting rapid photocrosslinked transparent ocular bandage that can withstand high intraocular pressures than compared to sutures. Briefly, a 3mm linear incision was created in the central cornea resulting in a gaping wound. A tunnel incision was created. A 3-mm trephine central incision was created. The burst pressure with saline infusion was measured using a manometer system. Components for the formation of nanoglue (40% D-MA and 60% HA-MA) were premixed and applied on corneal incisions. An argon green laser (325 nm) was applied for 20-30 seconds.

### Results

The components for the formation of nanoglue were rapidly cured, resulting in a transparent gel conforming to the shape of the wounds, avoiding leakage of the fluid. It is believed that the nanoglue strongly adhered to the cornea, especially compared to sutures. Figure 2 shows the testing apparatus and a photograph of the incision and includes inset Table 1 that showing the nanoglue withstood high intraocular pressure compared to sutures.

### Example 3. Optical and mechanical properties of nanoglue compared to sutures in in vivo wounded rat cornea.

### Materials and methods

In-vivo evaluation the rats were anesthetized with and pupils were dilated with tropicamide and 10% phenylephrine. A 2.75-mm keratome was used to create a full thickness central corneal incision. The blade was initially directed posteriorly, and after the penetration it was directed to the angle to avoid damaging the lens and its capsule. The incision was then either sutured (10-0 prolene or nylon) or glued with above mentioned nanoglue. Following the suturing/gluing, the corneal incision was confirmed to be closed with fluorescein and cobalt blue light.

Follow-up was performed 24, 72 hours, 7 days and 2 weeks following surgery. The rat corneas were imaged using optical coherence tomography (OCT) on day 7. The animals were clinically assessed for wound healing until day 14.

### Results

Following the suturing/gluing with nanoglue, the corneal incision was confirmed to be closed with fluorescein and cobalt blue light using standard techniques. The anterior chambers were formed within 24 hrs. in both the nanoglue and suture treated groups.

The corneal sealed with nanoglue showed rapid healing and promising results than compared to sutures which resulted in inflammation, neovascularization and corneal irregularity. There was less corneal thickening around the incision. Both sutured and glued corneas demonstrated some degree of fibrosis that can be seen as hyper-reflectivity in the OCT scans (Fig. 3).

Inflammation and neovascularization were more exclusively observed in sutured corneas at day 7. On day 7, the corneal laceration sutured with a single 10-0 suture had fibrosis and corneal edema, which was believed to show improper wound healing. The corneal laceration sealed with nanoglue had some fibrosis but less corneal edema; it also had excellent wound apposition.

In the nanoglue treated group, the gel sealed the wound till day 10 and the wound healed completely with no signs of infection or toxicity and inflammation.

On day 14, all the rats treated with nanoglue had complete resolution of the wounds with only minimal scarring, whereas the sutured corneas developed corneal neovascularization, inflammation and corneal irregularity (Fig. 4).

Additionally, the sutured and glued eyes demonstrate a normal anterior segment architecture with a formed anterior chamber, similarly to the control eye. Aqueous chamber was formed within in 24 hrs both in the case of glue and sutures. Inflammation and neovascularization was demonstrated in sutured cornea at POD7. In the case of glue treated incisions. The glue appears to seal the wound till day 10 and the wound heals completely with no signs of infection or toxicity and inflammation (Fig. 4).

### Example 4. Preparation of photocrosslinked, dendrimer-hyaluronic acid hydrogel with entrapped dendrimer-drug conjugates for subconjunctival injections for treating corneal inflammation and infections.

### Materials and methods

### Materials

Hydroxyl- and amine- functionalized ethylenediamine core generation four PAMAM dendrimers (G4-OH; diagnostic grade; 64 end-groups) were purchased from Dendritech Inc. (Midland, MI, USA). Dexamethasone (Dex), succinic anhydride (SA), *N,N'-*diisopropylethylamine (DIEA), trifluoroacetic acid (TFA), anhydrous dimethylformamide (DMF), dimethylacetamide (DMA) and 4-Pentenoic acid(Kosher) were purchased from Sigma-Aldrich (St. Louis, MO, USA). Hyaluronate thiol, MW 10k (10% degree of substitution) was purchased using custom synthesis service from creative PEGWorks (Chapel Hill, NC, USA). (Benzotriazol-1-yloxy)tripyrrolidino-phosphonium hexafluorophosphate (PyBOP) was purchased from Bachem Americas Inc. (Torrance, CA, USA). Cy5-mono-NHS ester was purchased from Amersham Biosciences-GE Healthcare (Pittsburgh, PA, USA). ACS grade DMF, dichloromethane (DCM), diethylether, hexane, ethyl acetate, HPLC grade water, acetonitrile, and methanol were obtained from Fisher Scientific and used as received for dialysis, purification and column chromatography. Dialysis membrane (MW cut-off 1000 & 2000 Da) was obtained from Spectrum Laboratories Inc. (Rancho Dominguez, CA, USA).

### Modification of Dex with a succinate

Dex (500 mg, 1.28 mmol) was dissolved in 10 mL of DMF/DMA (8:2) in a 50 mL round bottom flask (RBF) under nitrogen atmosphere and ice bath. Succinic anhydride (192 mg, 1.91 mmol) dissolved in 5mL of DMF/DMA (8:2) and 0.25 mL of TEA were added to it and the reaction mixture was stirred at 0°C for 3 h and then at room temperature for 48 h. Dexamethasone has three hydroxyl groups and the most reactive hydroxyl group is in 21-position. In order to avoid conjugation of linker to -OH groups at 11 and 17 positions of Dex, succinic anhydride of no more than 1.2 mole equivalents compared to dexamethasone was used in the reaction.

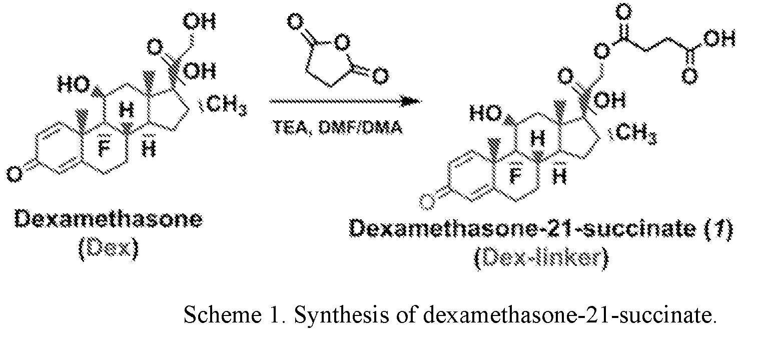

The completion of the reaction was monitored by thin layer chromatography (TLC), performed on silica gel GF₂₅₄ plates (Whatman, Piscataway, NJ), using ethyl acetate/methanol (90:10) as mobile phase. The reaction solvent was evaporated under reduced pressure and the crude product was purified with column chromatography by passing through silica gel using ethyl acetate:methanol (99.5:0.5) as eluent to get Dex-21-succinate (535 mg, >90% yield). The obtained purified product was characterized by using ¹H NMR spectroscopy with DMSO-*d₆* as the solvent and tetramethylsilane (TMS) used as internal standard.

### Synthesis of Dendrimer-Dex conjugate

Dex-21-succinate (Dex-Linker, 255 mg, 0.541 mmol) was dissolved in anhydrous DMF (5 mL) in a 50 mL round bottomed flask under nitrogen at 0°C, to which PyBOP (703.9 mg, 1.35 mmol) dissolved in DMF (5 mL) and DIEA (300 µL) was added, and the reaction mixture was allowed to stir for 1 hour in an ice bath. PAMAM G4-OH (505 mg, 0.036 mmol) dissolved in anhydrous DMF (10 mL) was added drop wise to the reaction mixture above, and stirred for 48 hours under nitrogen. The mixture of solvents was evaporated at 25°C under vacuum. The crude product was re-dissolved in DMF (20 mL) and subjected to dialysis in DMF (membrane MW cutoff = 2 kDa) for 48 hours, where the solvent was changed at least 6 times. The obtained solution was evaporated under reduced pressure at room temperature and the resulted sticky viscous solution was precipitated using cold ether twice to remove traces of DMF. The resultant product was re-dissolved in methanol and co-evaporated under reduced pressure and subjected to high vacuum treatment for overnight, to produce an off-white semi-solid dendrimer-triamcinolone conjugate (D-Dex, 630 mg). The resultant semi solid product was dissolved in ice cold DI water and dialyzed against DI water (membrane MWCO = 1 kDa) at 4°C for 5 hours by changing the water every hour to remove traces of DMF and methanol. The resultant water layer was lyophilized to get fluffy white powder of D-Dex (600 mg).

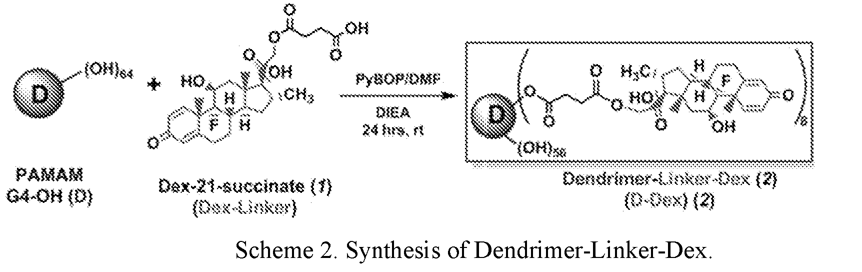

The D-Dex conjugates were characterized by ¹H NMR for drug loading and reverse-phase HPLC for purity.

### Modification of Dendrimer with an -ene- or -nor- group

We have also developed hydrogel formulations that can form flexible and injectable gels or corneal adhesive using photo-click thiol-ene chemistry. D-ENE or D-Nor were synthesized using single step coupling reactions between surface hydroxyl groups (-OH) of PAMAM dendrimers and the carboxylate moiety of 4-pentenoic acid or norborene acid. Using proton NMR, we estimated that approximately 28-29 molecules of 4-pentenoic acid were conjugated to one dendrimer molecule. The conjugates were readily soluble in water, PBS buffer and saline.

4-pentenoic acid (ENE, 60.0 mg, 0.58 mmol) was dissolved in 3 mL of anhydrous DMF in a 50mL RBF under nitrogen atmosphere at 0°C, to which PyBOP (560 mg, 0.88 mmol) dissolved in DMF (5 mL) and DIEA (300 µL) was added, and the reaction mixture was allowed to stir for 1 hour in an ice bath. G4-OH (550 mg, 0.04 mmol) was dissolved in 10 mL of anhydrous DMS and was added drop wise to the reaction mixture above, and stirred for 48 hours under nitrogen. The mixture of solvents was evaporated at 25°C under vacuum. The crude product was re-dissolved in DMF (20 mL) and subjected to dialysis in DMF (membrane MW cutoff = 1 kDa) for 24 hours, where the solvent was changed at least 4 times. The obtained solution was evaporated under reduced pressure at room temperature and the resulted sticky viscous solution was precipitated using cold ether twice to remove traces of DMF. The resultant product was dissolved in ice cold DI water and dialyzed against DI water (membrane MWCO = 1 kDa) at 4°C for 6 hours by changing the water every hour to remove traces of DMF The resultant water layer was lyophilized to get fluffy glassy-white powder of D-ENE (590 mg).

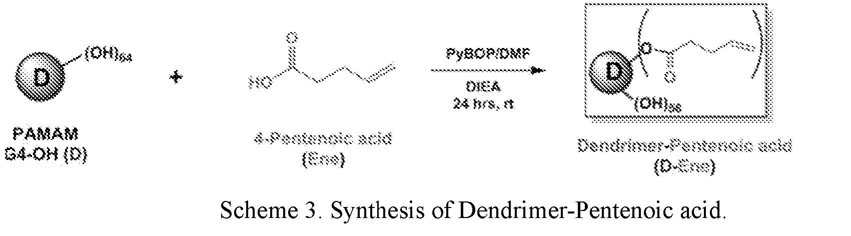

The D-Dex conjugates were characterized by ¹H NMR for drug loading and reverse-phase HPLC for purity.

### Modification of hyaluronic acid with a free thiol group

Sodium salt of HA (NaHA) was first converted to tetrabutylammonium salt (HA-TBA) to be able to solubilize in DMSO/DMF (20:80) for efficient thiolation reaction. To make HA-TBA, NaHA was dissolved in ultrapure water (UPH-DI H₂O) at 2% (w/w) and the ion exchange was done by adding DOWEX 50W proton exchange resin (Sigma, MO, USA) (3 g resin per 1g NaHA) with vigorous stirring for 5 h. The resin was filtered off using WHATMAN filter paper and the filtrate was titrated to a pH of 7.4 with TBA-OH. The resulting solution was lyophilized at -80°C to obtain off-white floppy solid, further the solid was dissolved in DI-H₂O and subjected to water dialysis (membrane MWCO = 2 kDa) to remove excess TBA-OH. The resultant water layer was again subjected to lyophilization and stored at -20°C until used. The ion exchange was confirmed using ¹H NMR analysis.

HA-TBA (615 mg, 0.027 mmol) was dissolved in 10 mL of anhydrous DMSO at 50°C in a 100mL RBF under nitrogen atmosphere, to which DCC (640 mg, 3.01 mmol) and DMAP (137.5 mg, 1.12 mmol) dissolved in 20mL of anhydrous DMF. A catalytic amount of hydroquinone was added to the reaction mixture to avoid formation of disulfides. 3-mercapto propionic acid (300 mg, 2.81 mmol) was dissolved in 10 ml of anhydrous DMF and added dropwise in to RBF and the reaction was stirred for 48 h under nitrogen atmosphere at room temperature. The reaction mixture was centrifuged in 50 mL centrifuge tubes to remove formed DCU and the solvent layer was dialyzed against DMF in 2KD MWCO dialysis tubing for 36 h under vacuum to remove unreacted compounds and soluble DCU. The obtained solution was evaporated under reduced pressure to obtain sticky semi-solid TBA-HA-SH which was dissolved in NaCl solution (0.5 g NaCl per 100mL of H₂O with catalytic amount of TECEP) and stirred for 20 mins and the solution was precipitated three times with cold acetone and then with cold ethanol five times to remove TECEP and excess salt. The resultant white sold was dissolved in DI H₂O and immediately frozen in liquid nitrogen and lyophilized at -80°C to obtain white power of HA-SH (370 mg).

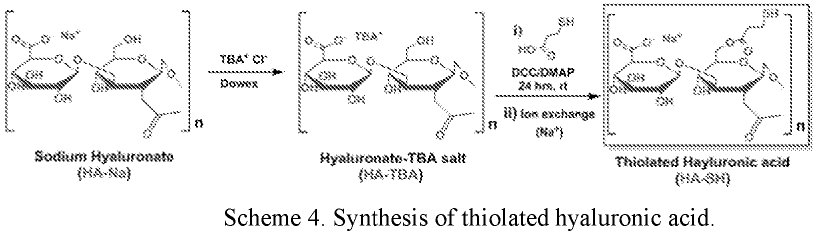

### Alternative Modification of hyaluronic acid with a free thiol group

Thiolated hyaluronic acid was synthesized with high degree of substitution as a thiol component for photoclick hydrogels. HA-SH was synthesized using a three-step process. In the first step the commercially available sodium salt of hyaluronic acid (HA) was converted to DMF/DMSO soluble tetrabutylammoninum (TBA) salt (HA-TBA) using the ion exchange method. In the second step, 3-(tritylthio)propionic acid was conjugated to the hydroxyl (-OH) groups of hyaluronic acid using a Steglich esterification reaction. We used trityl protected thiopropionic acid to avoid thioester and disulfide bonds formation. In the third step, we selectively de-protected the trityl group using 5% TFA in a DMF/DCM (1:5) mixture and in the presence of Et3 SiH to obtain a TBA-HA-SH intermediate. Then, TBA+ groups were fully exchanged using NaCl solution and the reaction mixture was washed with cold acetone and ethanol to remove the excess TBA, DTT and NaCl to yield hyaluronic acid bearing free - SH end groups.

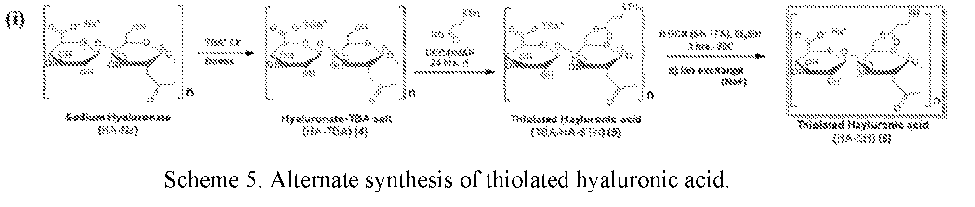

### Preparation of Formulation 2

The chemically crosslinked nanoglue formulation was formed by mixing HA-SH and D-Ene or D-Nor at a ratio of (50:50) with 0.005% photo initiator (Irgacure) using a dual barrel syringe and can be applied over the corneal incision. For curing a cobalt blue light or a flash light and be illuminated resulting in nanoglue photo crosslinking within 30 seconds (Fig. 1G).

### HPLC characterization of the conjugates

The purity of the dendrimer conjugates was analyzed by HPLC (Waters Corporation, Milford, MA) equipped with a 1525 binary pump, a 2998 photodiode array (PDA) detector, a 2475 multi-wavelength fluorescence detector, and a 717 auto sampler (kept at 4°C) interfaced with Empower software. The HPLC chromatograms were monitored at 205 (G4-OH) and 239 nm (Dex conjugated dendrimers) using PDA detector. The water/acetonitrile (0.1% w/w TFA) was freshly prepared, filtered, degassed, and used as mobile phase. A TSK gel ODS-80 Ts (250 X 4.6 mm, i.d., 5 µm) with TSK gel guard column were used for the study (Tosoh Bioscience LLC, Japan). A gradient flow was used with initial condition of 90:10 (H₂O/ACN) gradually changing the ratios to 70:30 (H₂O/ACN) until 20 min and then gradually increasing the acetonitrile percentage to 50% such that the ratios are 50:50 (H₂O/ACN) at 30 min then again decreasing the acetonitrile to 30 percent such that the ratios are 70:30 (H₂O/ACN) at 40 mins. The gradient was returned to initial conditions 90:10 (H₂O/ACN) in 50 min with flow rate of 1 mL/min for all conjugates.

### Photocrosslink dendrimer-hyaluronic acid hydrogel in a syringe

Injectable dendrimer-hyaluronic acid gel was prepared via thiol-ene photopolymerization by using the dendrimer component (D-ENE) and the hyaluronic acid component (HA-SH) in the ratio of 1:2 respectively. Briefly, 2% solutions of individual components were prepared in PBS and were stored on ice until mixed. D-Dex and free Dex solutions were prepared by dissolving D-Dex and free Dex in PBS such that 10 µL of the solution contains 1.6 mg of Dex in the form of D-Dex or free Dex. For each injections 20 µL of HA-SH solution, 10 µL of D-ENE solution, 10 µL of D-Dex or free Dex solution and 5 µL of photo-initiator (Irgacure 2959 (Ciba, Basel, Switzerland), 5 mg/mL in DMSO) were mixed in 0.5 mL eppendorf tubes. The hazy mixture solution was loaded in to 0.5 cc insulin syringe and placed under UV light for 5 min. In this formulation D-Dex or free Dex are physically entrapped in the injectable gel.

### Results

Generation four hydroxyl terminated PAMAM dendrimer (G4-OH) was used due to its low toxicity profile and near neutral surface charge, enabling non-specific retention and interactions in the tissue. A succinic acid spacer was used between the dendrimer and the conjugated drug, since the ester linkage enabled better drug release (Kambhampati SP, et al., Eur J Pharm Biopharm, Sep;95(Pt B):239-49 (2015)).

The structures after modifications or conjugations were confirmed with ¹H NMR spectra for the following reactions:
(i) Dex-Linker was characterized by peaks at 1.34, 1.36 and 1.87 ppm corresponding to -CH₂ protons of linker, and a peak at 12.22 ppm corresponding to carboxylic acid confirming the formation of Dex-Linker;
(ii) Dendrimer-Dex (D-Dex) was characterized by three peaks at 0.80, 0.88 and 1.49 ppm representing methyl protons (-CH₃) of Dex, a new peak at 4.02 ppm corresponding to modified methylene protons (-CH₂) of dendrimer, and peaks between 5.0 and 7.4 ppm corresponding to aromatic protons of Dex; based on the proton integration from NMR, approximately 7-8 molecules of Dex were conjugated to one dendrimer molecule. The conjugates were readily soluble in water, PBS buffer, and saline;
(iii) Dendrimer-ene (D-Ene) was characterized by new multiplet peaks around 4.97, 5.05, 5.80 ppm corresponding to H₂C= and =CHC alkene protons of 4-pentenoic acid, and a new peak at 4.02 ppm which corresponds to modified methylene protons (-CH₂) of dendrimer. Based on the proton integration, approximately 15 molecules of 4-pentenoic acid were conjugated to one dendrimer molecule. The conjugates were readily soluble in water, PBS buffer, and saline; and
(iv) HA-TBA salt was characterized by a new multiplet peak between 0.85 to 0.87 ppm corresponding methyl (-CH₃) protons of TBA and two new peaks from 1.23 to 1.59 ppm corresponding to methylene (-CH₂) protons of TBA. Thiolated HA was characterized by the absence of the characteristic peaks of TBA.

Reverse phase HPLC confirmed the purity of the Dex-linker and the Dendrimer-Dex (D-Dex) conjugate. The hydrophobic free Dex eluted at 22.4 min, whereas the Dex-linker eluted at 27.6 min (with no trace at 37.1 min). At similar HPLC conditions, a broad peak at 32.8 min was observed for D-Dex conjugate (monitored at 240 nm), which was different from that for the starting dendrimer (retention time 15 min). The conjugate was largely pure, as no 'characteristic' peaks related to free Dex or Dex-linker was observed. The broad peak of D-Dex conjugate can be attributed to the high loading (~8 molecules per dendrimer), resulting in some non-polar characteristics.

Figure 6 shows a diagram of the formation of dendrimer-hyaluronic acid gel entrapping dendrimer-dex conjugates. D-Ene was used to anchor the linear thiolated hyaluronic acid (HA-SH). Upon pushing the piston, jelly solution was released from the needle tip, confirming the formation of injectable, transparent gel.

### Example 5: Rheological properties and morphology of injectable hydrogels.

The formation of gel and crosslinking kinetics were assessed using time sweep measurements. Before UV irradiation (till ~60 s) and until 40 seconds after irradiation, the solution had a low viscosity (Fig. 5A), suggesting an easily injectable solution. After UV irradiation at 60 s, storage modulus (G') increased, the loss modulus (G") stabilized, with the crossover point (G'>G") occurring at ~160 s (i.e. 100 s after UV treatment), suggestive of gelation via thiol-ene click within the loaded polymer solution over this period (Figure 3A). This suggests that gelation occurred within little ~1 min of UV exposure and the gels reached a storage modulus of ~1 kPa. The gelation time was unaffected with the incorporation of D-Dex in the prepolymer solution. The frequency sweep (0.01-10 Hz, within LVER) measurements after gelation were conducted at 37°C under a hydrated environment, to assess G', G" and complex viscosity (|η*|) (Figs. 5B and 5C). For all injectable gels (with or without D-Dex), G" was always lower than G' (G'»G"), and was independent of frequency, suggestive of a stable, viscoelastic, crosslinked network (Fig. 5B). Incorporation of D-Dex did not significantly change the rheological properties of the injectable gel (without D-Dex G'- 148.4±0.5 Pa, G" - 5.2±0.1 Pa, with D-Dex G' - 216.5±0.9 Pa, G" - 8.2±0.08 Pa). The magnitude of the complex viscosity (|η*|) decreased with increasing frequency, indicating that the injectable gel was shear thinning (Figs. 5C and 5D). The shear thinning was attributable to HA in the gel system, as observed in Healon (an injectable HA gel). There was a small increase (~22.5%) in viscosity in D-Dex incorporated gels suggestive of a small D-Dex induced filler effect.

SEM imaging was used to assess the surface morphology of the injectable gels with and without D-Dex. The HMDS dehydrated gels showed uniformly dense structures with striations in both conditions (with and without D-Dex) (Fig. 5F). Interestingly, in the dried form, D-Dex appears to be incorporated into the injectable gel as precipitates (Fig. 5F). Dehydration of gel samples exhibited significant reduction in volume (~45% reduction) from the hydrated state. It is likely that the removal of water causes formation of dense structure and D-Dex precipitation in D-Dex incorporated gels. The cross sections of the FITC-labeled hydrogels were imaged under confocal microscope to investigate the crosslinked morphology and pore density. The cross sections demonstrated similar crosslinked architecture forming pores with size ranging from 60 nm to 100 µm in both the naïve hydrogel and in D-Dex incorporated gel (Fig. 5G).

### Example 6. In vitro release of Dex from Dendrimer-Dex (D-Dex) Conjugate and dendrimer-hyaluronic acid hydrogel.

### Materials and methods

The D-Dex conjugate was prepared and characterized as described in Example 4. A succinic acid spacer was used between the dendrimer and the conjugated drug, and the ester linkage enabled release of the drug from the D-Dex conjugate.

The release of Dex from the D-Dex conjugate was characterized in simulated vitreous humor [Hanks balanced salt solution with 0.03% sodium hyaluronate (Lifecore biomedical, MN, USA) and 0.1% TritonX (Sigma, MO, USA)] as a stabilizer and surfactant to reduce released Dex settling. A concentration of 3mg/mL was maintained in water bath at 37°C equipped with shaker. At appropriate time points, 200µL of solution was withdrawn from the incubation mixture, frozen in liquid nitrogen and lyophilized. To this lyophilized powder, 400µL of 50:50 (DCM:EtOAc) was added and sonicated for 10 min and centrifuged at 10,000 rpm for 5 min at 4°C. The supernatant was collected and the solvent was evaporated by nitrogen flush and reconstituted with 200µL of 50:50 H₂O:ACN and subjected to HPLC analysis. The percent of released Dex from D-Dex was quantified using the calibration graph.

Swelling and degradation of injectable gel were assessed in physiological relevant fluids such as phosphate buffer saline (PBS 1X, pH 7.4) and citrate buffer (pH 5). For stability studies, gel pellets with known amounts of D-Dex or free Dex were made using the circular crevice in the caps of the 2 mL eppendorf tubes (diameter = 8.0 mm and thickness 4 mm). The pre-weighed pellets (n=3, for each group) were placed in a 12-well plate containing 1 mL of buffer and incubated at 37°C. At discrete time points the pellets were carefully lifted using a spatula and the excess moisture blotted out using kimwipes and weighed. The swelling ratio was calculated using the equation (W*_{c}* / W*ᵢ*)×100, where W*_{c}* is the current swollen weight and W*ᵢ* is the initial weight of the gel. The degradation rate was calculated using the equation ((W*ᵢₛ* - W*_{cd}*)/ W*ᵢₛ*) ×100, where W*ᵢₛ* is the initial swollen weight and W*_{cd}* is the cured decreased weight.

The release of D-Dex and free Dex from the injectable gel was analyzed by incubating the gel pellets in 8 mL scintillation vial containing 5 mL of SAH solution. At particular time points 100 µL of samples were withdrawn and analyzed using HPLC. The percent of released free Dex from D-Dex was quantified using the calibration graph.

### Results

The results are shown in Figures 7A-7E. Figure 7A is a HPLC chromatogram of D-Dex (32.8 min), Dex (22.4 min) and Dex-Linker (27.6 min) monitored at 239 nm.

Figure 7B is a graph of drug release from D-Dex conjugates in simulated tear fluid. Figure 7C is a graph of D-Dex and Free Dex release from injectable gel formulations at pH 7.4 and 5, respectively. Release profiles at earlier time points show burst release.

Figure 7D is a graph of the swelling and degradation profile of injectable gel in pH 7.4 and 5, respectively. Figures 7E and 7F are graphs of the weight changes depicting the swelling properties of the gel.

### Example 7. In vivo biodistribution in rat cornea of dendrimer molecules entrapped in the dendrimer-hyaluronic acid crosslinnked gel

### Materials and methods

### Tagging dendrimer molecules with a fluorescent label

Fluorescently labeled G4-OH dendrimer was synthesized and characterized as described in Lesniak WG, et al., Mol Pharm., 10(12):4560-4571 (2013). Briefly, generation four hydroxyl-terminated PAMAM dendrimer (D) was modified with reactive amine surface end groups, which was further reacted with N-hydroxysuccinimide monoester Cy5 dye to generate the D-Cy5 conjugate.

### Corneal alkali burn model

All procedures involving animals conformed with the IACUC research guidelines by the Association for Research in Vision and Ophthalmology Statement for the use of Animals in Ophthalmic and Vision Research and Johns Hopkins University. A total of 57 Lewis rats (7 to 8 weeks of age) were obtained from Harlan Laboratories Inc. (Frederick, MD). All animals weighed between 150 and 200 g, and they were housed at constant temperature (20 ± 1°C) and humidity (50 ± 5%). They were fed standard rat chow and had access to water *ad libitum.* All procedures and tests were performed under general anesthesia with intramuscular injection of ketamine 0.9% (Bio-niche Pharma, Lake Forest, IL, USA)/xylazine 0.1% (Phoenix Pharmaceuticals, St. Joseph, MO, USA) and topical proparacaine 0.5% (Sandoz, Holzkirchen, Germany). Corneal alkali burn was generated by applying 0.5N NaOH on cornea. Briefly, sample discs SS-033 0.5 cm in diameter (WESCOR, Logan, Utah, USA) were cut in to 4 quadrants. The quadrant was soaked in 0.5N NaOH for 10 seconds and then placed on central cornea for 15s. The burn area and the conjunctival sac were immediately irrigated with 30 mL of BSS solution using an eyedrop bottle.

### Administering to rat eyes

D-Cy5 was dissolved in PBS (250 µg D-Cy5 in 10uL) and integrated into 40 µl of an injectable gel system as described in example 4 and were loaded in to 0.5 mL 30G insulin syringes, followed by UV light exposure for 3 mins until gel was formed. The D-Cy5 gels were injected into subconjunctiva, forming blebs. Seven days post injection of D-Cy5, the rats were euthanized using a lethal dose of sodium pentobarbital (Lundbeck, Deerfield, IL, USA) and the eyes were collected.

### Immunohistochemical analysis

The eyes were enucleated and were washed in ice cold PBS for 5 mins. The eyeballs were fixed in 4% PFA in 5% sucrose solution for 5 hours and then subjected to treatment with sucrose gradient. The eye balls were frozen in a 20% Sucrose/optimum cutting temperature (OCT) in a 1:2 ratio, respectively, using dry ice in isopentane. Cryoblocks were stored at -80°C until 20-µm sections were cut using cryostat (microm, Walldorf, Germany). Four sections from each cryoblock were used for image analysis. The sections were incubated in rabbit anti-ionized calcium binding adapter 1 molecule (Iba-1; Wako Chemicals, Richmond, VA, USA), which is a macrophage cell marker; and then a goat anti-rabbit-Cy3 secondary antibody (Life Technologies, Grand Island, NY, USA) and isolectin GS-IB4 AF488 (Thermo Fisher, MA) for stroma and blood vessels were applied. The corneal and iris part of the sections were analyzed using a confocal microscope (model 710 unit; Carl Zeiss, Inc., Thornwood, NY, USA). Excitation and emission wavelengths and laser settings were identical for all tissues. Z-stacks of sections were taken and collapsed to give an image through the depth of the whole section.

### Results

Iba-1 was used as a marker for corneal macrophages. In a normal eye, Iba-1+ cells were found in minimal amounts in the central cornea region (Fig. 8A). Following alkali burn to the central cornea, an increase in macrophage infiltration (Iba-1+) was noted in the corneal stroma and the epithelial layers (Fig. 8B). The burn injury also resulted in a significant increase in corneal thickness which is a finding clinically consistent with post-alkali burn keratitis (corneal inflammation). In order to demonstrate both targeting and one-week retention of dendrimers released from the subconjunctival injectable gel, we used fluorescently labelled dendrimer (D-Cy5). In order to avoid tissue auto-fluorescence we used a near IR imaging agent (Cy5) covalently attached to dendrimer using previously established procedures in our lab (S.P. Kambhampati, et al., Intracellular delivery of dendrimer triamcinolone acetonide conjugates into microglial and human retinal pigment epithelial cells, European Journal of Pharmaceutics and Biopharmaceutics 95 (2015) 239-249, W.G. Lesniak, et al., Biodistribution of fluorescently labeled PAMAM dendrimers in neonatal rabbits: effect of neuroinflammation, Molecular pharmaceutics 10(12) (2013) 4560-4571). Seven days post subconjunctival injection of D-Cy5 gels, the imaging studies demonstrated pathology dependent biodistribution: D-Cy5 released from the gels were found co-localized and retained in infiltrating macrophages in the central cornea in the alkali burn group (Figure 8B), whereas in normal eyes, no D-Cy5 signals were elicited (Fig. 8A) suggesting that dendrimer biodistribution is restricted to inflamed and pathologic tissues/cells. Additionally, alkali burn causes activation and infiltration of macrophages in the iris and D-Cy5 was also found co-localized in macrophages in the near vicinity of the iris blood vessels in inflamed tissues only (data not shown). The pathology dependent biodistribution can be attributed to a combination of factors such as dendrimer properties (size and surface charge), disruption of barriers in pathological tissue and altered properties of activated macrophages (Nance, E., et al., Nanoscale effects in dendrimer-mediated targeting of neuroinflammation, Biomaterials 101 (2016) 96-107).

### Example 8: In vivo subconjunctival treatment with dendrimer-hyaluronic acid gel delivering dendrimer-dexamethasone (D-Dex) following corneal alkali burn in rats.

### Materials and methods

Corneal alkali burn was performed on rats as described in Example 7.

### Administering gel treatments

After alkali burn, the animals were randomized for treatment in one of the following three groups: (i) Dendrimer-Dexamethasone conjugates entrapped in injectable, dendrimer-hyaluronic acid photocrosslinked gel with a minimal amount of free dexamethasone (D-Dex group, n=10), (ii) free dexamethasone in injectable, dendrimer-hyaluronic acid photocrosslinked gel (free-dex group, n=10), and (iii) no drug, only injectable, dendrimer-hyaluronic acid photocrosslinked gel, n=10. Both D-Dex and free-Dex groups were treated with equivalent Dex basis present in both D-Dex and free Dex formulations (1.6 mg of Dex/eye). Given that the D-Dex conjugates did not release enough dexamethasone in the first few days after its administration, a decision was made to augment it with a bolus of 5% of the total dose of free dexamethasone per dose (i.e.: 1.6mg dexamethasone conjugated to the dendrimer gel and an additional dose of 0.16mg of free dexamethasone). The free dexamethasone group received a total of 1.6mg of free dexamethasone. It is unlikely that the small additional dose of the free drug in the dendrimer group (0.16mg) had a therapeutic effect beyond the first few hours after injection. All forms of drugs were incorporated into injectable gel formulations (it is the same gel formulation as of example 4). The gel components are premixed and loaded in to the syringe and crosslinked to form gel using UV exposure. At the time of injection the components are in the form of gel) and injected subconjunctivally using 30-gauge, 0.5 cc insulin syringes. The total volume administered did not exceed 40 µL.

### Clinical and molecular-level evaluations

Animals were assessed at baseline and at the following post-exposure time points: 24 hours, 72 hours, and 7 days. A subset of the animals was also followed up to 14 days.

The central corneal thickness (CCT) and stromal structure analysis were done using anterior chamber optical coherence tomography (OCT) (Bioptigen Envisu R-class OCT, Bioptigen, NC, USA).

Changes in intraocular pressure (IOP) were measured using a handheld tonometer (Icare LAB tonometer, Icare, Finland). The settings R for rat were chosen and three individual measurements of IOP were made for each eye for accuracy.

Corneal opacity was scored by grading the degree of transparency as previously reported by Larkin DF, et al., Clin Exp Immunol., 107:381-391 (1997). The estimated area of neovascularization (NV) was assessed by estimating the radial penetration of the neovascular vessels and their extent in degrees, and by assuming a mean corneal diameter of 2.5mm, so that the maximal area of NV was no more than 2.52π mm² (=19.63 mm²).

For immunohistochemical analysis, the corneas (n=3, for each group) were washed in cold PBS and processed as described in Example 7, Immunohistochemical analysis. Stained sections were imaged under confocal microscope. The corneal macrophages in the central cornea were counted manually.

For cytokine expression analysis, the corneas (n=10, for each group) were snap frozen in liquid nitrogen and homogenized carefully into tissue powder using pre-cooled (in liquid nitrogen) porcelain mortar and pestles. The total RNA was purified with TRIzol reagent (Life Technologies, Grand Island, NY) following the manufacturer's instruction. 3 µg of total RNA was reverse-transcribed to cDNA using High-capacity cDNA Reverse Transcription System (Life Technologies) according to the manufacturer's instructions. qRT-PCR was performed with fast SYBR Green Master Mix (Life Technologies) by a StepOnePlus Real-Time PCR System (Life Technologies). Rat GAPDH was used to normalize the expression levels of target gene and calculated by the comparative cycle threshold Ct method (2^-(ΔΔCt)).

### Results

Central corneal thickness (CCT): There were no baseline differences in CCT between the groups (D-Dex vs. free-Dex: p=0.4; D-Dex vs. positive controls: p=0.67; free-Dex vs. positive controls: p=0.69). CCT was increased in all groups at POD 1 and 3 (see Figs. 9, 10A and Table 3), as expected. By POD 7 there was a trend for improvement in corneal thickness in all groups, but it was more pronounced in the D-dex group. Mean corneal thickness at POD14 was thinnest in the D-dex group. A comparison of the corneal thickness between the D-dex group and the free-dex group showed that the CCT was thinner in the former at POD3 (p=0.04) and POD7 (p=0.009) (see Figs. 9, 10A and Table 3). For the comparison of the D-dex group and the positive controls, CCT was thinner for the former at POD3 (p=0.01), POD7 (p=0.001), and POD14 (p=0.01). Treatment with steroids leads to mitigation of intraocular and corneal inflammation with a resultant decrease in corneal thickness. As shown here, the D-Dex group had the most favorable outcome in terms of resolution of corneal edema following alkali burn.

Intraocular pressure: As demonstrated in Figure 10B and in Table 3, there was no baseline differences in IOP between any two of the three groups (d-dex vs. free dex p=0.25; d-dex vs. non-treated: p=0.3; and free-dex vs. non-treated: p=0.89). Statistically significant differences were first noted at POD3: the mean IOP in the d-dex group was 10±0.2 mmHg, whereas in the free-dex group it was 12.14±0.63 (p=0.02); the mean IOP in the untreated group was 11.91±1.28 (p=0.04 for the comparison with the d-dex group). By POD7, these differences became more apparent with the d-dex group having a mean IOP of 11.33±0.62mmHg, whereas the free-dex group had a mean IOP of 14.45±0.5mmHg (p=0.001). At POD14 the d-dex group had a mean IOP of 10.9±0.66mmHg, similar to its baseline IOP, whereas the free-dex group had a mean IOP of 19.38±1.8mmHg (p<0.001) (see Fig. 10B and Table 3). Of note, neither one of the steroid treated groups had a clinically significant elevation in IOP, however the D-Dex group had a more favorable outcome with a more modest increase in IOP and no apparent IOP spike. This can be attributed to the slow steroid release profile of this particular dendrimer-dexamethasone formulation. Avoiding an IOP spike or persistent elevation in IOP is a significant advantage in clinical practice.

Estimated area of neovascularization: Neovascular vessels appeared in all groups no earlier than POD3. As shown in Figures 10C, 4, and Table 3, the area occupied by neo-vessels remained relatively stable in the d-dex group with a mean area of 2.5±0.32 mm² at POD7, compared to the mean area in the free-dex group: 3.32±0.34 (p=0.009). Neovascularization is a later sequela in the inflammatory cascade. Inhibition of inflammation at earlier stages could explain the difference seen here, with D-Dex having the best outcome.

Corneal opacity score: Median opacity scores were all zero at baseline for all studied groups. The corneas lost their transparency as soon as POD1, however, the positive control group never recovered, as shown in Figures 10D, 4, and Table 3- at POD14 the control group had a mean opacity score of 2.5 (range: 1-4). At POD14 the untreated group had a mean opacity score of 2.5 (range: 1-4). Statistically significant differences were observed between the groups as early as POD3. The median opacity score for the d-dex group was 2.5 (range:0-4), whereas for the free-dex group it was 3.5 (range: 1-3, p<0.001). This difference remained statistically significant at POD7 (p=0.006) and at POD14 (p=0.008). Corneal opacity in the constellation of alkali burn is a result of corneal inflammation and edema. The D-Dex group had the best outcome in terms of clinically assessed corneal opacity (see Figs. 10D, 4, and Table 3). This is another measure of the enhanced efficacy of the D-Dex conjugate in the treatment of corneal inflammation.

Immunohistochemistry and confocal microscopy: Immunohistochemistry and high resolution imaging using confocal microscopy was used at POD 7 and 14 in order to qualitatively assess the number of macrophages present in the central cornea. This measure provided an additional indirect estimate of the ability of the different treatments to decrease tissue inflammation. As previously explained in the biodistribution section, alkali burns cause structural damage and macrophage infiltration of the cornea.

The positive control group, that was treated with a placebo gel showed accumulation of macrophages in the central cornea at POD7, similar to what was seen in corneas exposed to alkali burn that had not been given any steroid treatment whatsoever. A persistent Iba-1 positive infiltrate (macrophages) was observed at POD 14 in these positive controls. We also observed some improvement in central corneal architecture that can be attributed to the natural healing process in rat model (Fig. 11, right panel). The Free-Dex gel group also demonstrated an Iba-1 positive cellular infiltrate (macrophages) of the central corneas at POD7, however it did partially resolve by POD14, this can be attributed to the therapeutic activity of free-Dex released from the subconjunctival gel (Fig. 11, middle panel). The best results in terms of macrophage depletion were seen in the D-Dex group, in which the lowest number of infiltrating cells was identified at POD7. By POD14, that infiltrate had almost completely resolved (Fig. 11, left panel).

Evaluation of inflammatory cytokine production using RT-PCR: Figures 12A-12E show the expression of inflammatory cytokines after different treatments. In order to further characterize the response to treatment in terms of amelioration of inflammation, inflammatory cytokines were assessed at POD7 and POD14. It is widely reported that alkali burn results in elevation of inflammatory cytokines (TNF-α, IL-1β, IL-6, IL-8 and VEGF) both in acute and chronic phases. At POD7, IL-6 mRNA levels were significantly lower in the D-Dex group when compared to positive controls (mean ± SEM 0.7±0.13 vs. 9.8±3.53, p<0.001) (Fig. 5); this was also true for the comparison of IL-6 levels in the free-Dex group compared to the positive controls (3.0±0.3 vs. 9.8±3.53, p<0.001). MCP-1 mRNA levels were significantly lower at POD7 in the D-Dex group compared to the positive controls (0.7±0.13 vs 4.0±1.24, p<0.001); however, this was not true for the comparison of the free-Dex group to the positive controls (p=0.259) (Figure 12). At POD14, both D-Dex and free-Dex group had benefited from treatment in comparison to the positive controls, as shown by the mRNA levels of MCP-1: 12.5±5.49 and 15.4±3.22, respectively vs. 98.7±15.42 in the positive control group (p<0.001 for both comparisons). Of note, at POD14 the mRNA levels of VEGF were only significantly lower in the D-Dex group when compared to the positive controls (6.3±1.02 vs. 27.2±5.66, p<0.001; p=0.004 for the comparison between free-Dex and the positive controls) (Fig. 12). This difference in mRNA for VEGF may explain why the D-Dex group had less corneal neovascularization at POD14 compared to the other groups. Overall, the results of the cytokine analysis are consistent with the clinical results of the present invention suggesting that the D-Dex group had the best outcome in terms of corneal inflammation resolution. This anti-inflammatory activity is highly beneficial in reducing the chances of graft failure in corneal transplantation surgeries.

Conclusions: Corneal inflammation is an important pathological event implicated to play a crucial role in many diseases progressing to their advance stages by disrupting normal corneal homeostasis. Topical steroids are beneficial in reducing leukocyte/macrophage recruitment but required to be dosed frequently which may result in corneal toxicity and melting (M.D. Wagoner, Chemical injuries of the eye: current concepts in pathophysiology and therapy, Survey of ophthalmology 41(4) (1997) 275-313; S. Den, et al., Efficacy of early systemic betamethasone or cyclosporin A after corneal alkali injury via inflammatory cytokine reduction, Acta Ophthalmologica Scandinavica 82(2) (2004) 195-199; P.C. Donshik, et al., Effect of topical corticosteroids on ulceration in alkali-burned corneas, Archives of Ophthalmology 96(11) (1978) 2117-2120). As shown in the present invention, therapies aimed at targeting the very cells responsible for inflammation and delivering steroids in a sustained manner, which can be administered at the time of surgery are a viable option. In the present invention, we used mild rat alkali burn injury as a model for corneal inflammation. The intrinsic targeting ability of PAMAM G4 hydroxyl dendrimers to localize in activated corneal macrophages is utilized to develop a sustained, targeted and intracellular delivery of dexamethasone to attenuate corneal inflammation. The D-Dex conjugate with a payload of ~20% was highly soluble in aqueous solutions. The conjugates demonstrated improved anti-inflammatory activity (~1.6 fold at 10-fold lower concentration than that of free dexamethasone) in LPS activated macrophages in vitro.

To extend and sustain the bioavailability of D-Dex, we developed an injectable gel system based on dendrimer and hyaluronic acid, crosslinked thiol-ene click photo chemistry. The gel formulations possess viscoelastic properties, are easily injectable and provide a sustained release of D-Dex. The dendrimers released from the injectable gel after subconjunctival administration targets and co-localizes in activated macrophages in central cornea with alkali burn. A single subconjunctival injection of D-Dex incorporated gel lead to prolonged efficacy for a period of 2 weeks. The D-Dex gel treatment demonstrated better outcomes such as reduced central corneal thickness, improved corneal clarity with no signs of elevation of intraocular pressure. The pharmacodynamics effect of D-Dex gel treatment attenuating corneal inflammation was demonstrated by significant reduction in macrophage infiltration in to central cornea and suppressed pro-inflammatory cytokines production compared to free drug.

The compositions of the present invention and their use disclosed herein support the concept that dendrimers are effective treatment vehicles in inflammatory disorders of the cornea. The compositions of the present invention and their use are also unique in the route of administration for the dendrimer-gel formulation- subconjunctival. This route is clinically accessible, does not necessitate an expensive resource such as an operating room, and the potential space can allow the administration of a relatively large volume of a drug. The delivery of a drug reservoir frees the patient of the need for repeat instillation of a topical drop and may improve compliance and outcome. Despite of the recent development of depot drugs for the posterior segment of the eye, to date there is no commercially available drug specifically designed to provide long standing drug delivery to the cornea. The system depicted in the present invention is specifically designed to deliver drugs to the anterior segment of the eye and has been shown to be efficacious in treating corneal inflammation.

### List of tables:

**Table 1: corneal opacity grading**

| **Opacity grade** | **Degree of transparency** |
|---|---|
| 0 | Transparent |
| 1 | Minimal loss of transparency |
| 2 | Iris vessels visible |
| 3 | Pupil outline visible |
| 4 | Pupil outline obscured |

**Table 1: Corneal opacity scores.** The degree of corneal transparency as a measure of efficacy after administration of subconjutival D-Dex, Free Dex or placebo. The grading measures were adopted from Larkin et al. (Identification and characterization of cells infiltrating the graft and aqueous humour in rat corneal allograft rejection, Clinical & Experimental Immunology 107(2) (1997) 381-391).

**Table 2: Primer sequences**

| **Primer** | **Segment** | **Sequence** |
|---|---|---|
| GAPDH (Glyceraldehyde 3-phosphate dehydrogenase) | Forward (5'-3') | GCAAGAGAGAGGCCCTCA (SEQ ID NO: 1) |
| | Reverse (3'-5') | |
| | | TGTGAGGGAGATGCTCAGTG (SEQ ID NO: 2) |
| TNF-α (Tumor necrosis factor-α) | Forward (5'-3') | TCAGTTCCATGGCCCAGAC (SEQ ID NO: 3) |
| | Reverse (3'-5') | |
| | | GTTGTCTTTGAGATCCATGCCATT (SEQ ID NO: 4) |
| IL-1β (Interleukin-1β) | Forward (5'-3') | CACCTCTCAAGCAGAGCACAG (SEQ ID NO: 5) |
| | Reverse (3'-5') | |
| | | GGGTTCCATGGTGAAGTCAAC (SEQ ID NO: 6) |
| IL-6 (Interleukin-6) | Forward (5'-3') | AAAGAGTTGTGCAATGGCAATTCT (SEQ ID NO: 7) |
| | Reverse (3'-5') | |
| | | CAGTGCATCATCGCTGTTCATACA (SEQ ID NO: 8) |
| MCP-1 (monocyte chemoattractant protein -1) | Forward (5'-3') | CTATGCAGGTCTCTGTCACGCTTC (SEQ ID NO: 9) |
| | Reverse (3'-5') | |
| | | CAGCCGACTCATTGGGATCA (SEQ ID NO: 10) |
| VEGF (Vascular endothelial growth factor) | Forward (5'-3') | GGCTTTACTGCTGTACCTCC (SEQ ID NO: 11) |
| | Reverse (3'-5') | |
| | | CAAATGCTTTCTCCGCTCT (SEQ ID NO: 12) |

**Table 3 - Summary of clinical outcomes**

| **Outcome measure** | **Group** | **Baseline** | **POD1** | **POD3** | **POD7** | **POD14** |
|---|---|---|---|---|---|---|
| **CCT** (µm) | D-Dex | 122.08±28 .17 | 288.58±72 .27 | 307.46±1 28.98 | 165.54±68. 64 | 174.9±115.1 5 |
| | Free-Dex | 142.86±8. 68 | 304.95±87 .23 | 357.71±1 15.58 | 229.57±91. 54 | 226.57±63.6 7 |
| | Positive controls | 132.8±20. 09 | 291.9±38. 67 | 370.3±92. 10 | 250.6±79.3 7 | 286.83±156. 57 |
| **Opacity score** | D-Dex | 0(0) | 2(3) | 2.5(2) | 1(1) | 0(0) |
| | Free-Dex | 0(0) | 3(3) | 3.5(1) | 1(1) | 1(2) |
| | Positive controls | 0(0) | 3(3) | 4(1) | 2.5(1) | 2.5(3) |
| **Estimated area of NV (mm²)** | D-Dex | 0±0 | 0± | 2.5±1.57 | 2.21±2.53 | 3.43±4.2 |
| | Free-Dex | 0±0 | 0±0 | 3.32±1.65 | 3.8±2.89 | 43.32±5.46 |
| | Positive controls | 0±0 | 0±0 | 4.03±1.02 | 7.74±3.02 | 8.29±5.4 |
| **IOP (mmHg)** | D-Dex | 10.29±1.7 3 | 10.63±2.0 2 | 10.00±0.9 8 | 11.33±3.03 | 10.90±2.08 |
| | Free-Dex | 11.36±1.8 4 | 10.73±2.0 7 | 12.14±2.9 3 | 14.45±2.32 | 19.38±3.89 |
| | Positive controls | 11.23±1.5 4 | 11.59±2.9 7 | 11.91±6.0 2 | 16.77±5.31 | 17.50±6.32 |

**Table 3: Summary of clinical outcomes after subconjutival treatment of D-Dex, free-Dex and placebo (Positive control) gels.** All results are displayed as mean±standard deviation except for the opacity scores that are displayed as median (inter-quartile range). POD - post operative day; CCT - central corneal thickness; NV - neovascularization; IOP - intraocular pressure.

## Claims

1. A composition comprising:
generation 2-10 poly(amidoamine) (PAMAM) dendrimers comprising one or more terminal hydroxyl groups and one or more photo-crosslinkable groups;
bioadhesive polymers comprising one or more photo-crosslinkable groups; and
a photoinitiator;
wherein the dendrimers and the bioadhesive polymers are crosslinked to each other through their respective photo-crosslinkable groups upon exposure to one or more external stimuli to form a nanoglue within a tissue, and
wherein the external stimuli is ultraviolet radiation, cobalt blue light, argon laser, or visible light.

2. The composition of claim 1, wherein the composition further comprises a therapeutic, prophylactic, or diagnostic agent conjugated to the dendrimers.

3. The composition of claim 2, wherein the dendrimers are conjugated to an agent selected from the group consisting of anti-inflammatory drugs, anti-infective agents, anti-glaucoma agents, agents that lower intraocular pressure (IOP), anti-angiogenesis agents, and growth factors.

4. The composition of claim 2, wherein the dendrimers are conjugated to a diagnostic agent selected from the group consisting of paramagnetic molecules, fluorescent compounds, magnetic molecules, radionuclides, x-ray imaging agents, and contrast media.

5. The composition of claim 2, wherein the dendrimers are conjugated to a therapeutic agent selected from the group consisting of dexamethasone, betamethasone, triamcinolone, prednisone, prednisolone, methylprednisolone, cortisone, hydrocortisone, and other steroids and non-steroidal anti-inflammatory drugs (NSAIDS).

6. The composition of claim 1, wherein the dendrimers are generation 2-6 poly(amidoamine) (PAMAM) dendrimers; optionally wherein the dendrimers are generation 4, 5 or 6 PAMAM dendrimers.

7. The composition of claim 1, wherein the bioadhesive polymers are selected from the group consisting of hyaluronic acid, chitosan, alginate, agarose, carboxymethylcellulose, hydroxymethylcellulose, methylcellulose, cellulose, polyalkylene oxide, poly(acrylic acid), poly(hydroxyethyl methacrylate), poly(vinyl pyrrolidone), poly(vinyl alcohol), pullulan, and derivatives thereof.

8. The composition of claim 1, wherein the bioadhesive polymers are hyaluronic acid or derivatives thereof.

9. The composition of claim 1, wherein the photo-crosslinkable groups are selected from thiol groups and maleimide groups; amine groups and aldehyde groups; amine groups and carboxylate groups; methacrylate groups; and acrylate groups.

10. A composition for use in a method of sealing tissue in an individual in need thereof, the method comprising:
administering to a tissue of the individual in need thereof the composition of any of claims 1-9; and
crosslinking the dendrimers and bioadhesive polymers within the tissue by exposure to the one or more external stimuli to form a nanoglue,
wherein the external stimuli is ultraviolet radiation, cobalt blue light, argon laser, or visible light.

11. The composition for use according to claim 10, wherein the composition is administered by injection.

12. The composition for use according to claim 10, wherein the composition is administered to the surface or compartment of an eye (subconjunctival, intracameral, supra-choroidal, topical, intravitreal or subretinal), or adjacent tissue; optionally
wherein the composition is administered subconjunctivally as a gel for sustained treatment of ocular/corneal inflammation; further optionally
wherein the method is for the treatment of alkali/acid burns, dry eye, corneal inflammation, corneal infections, iritis, or uveitis.

13. The composition for use according to claim 10, wherein the composition is:
a) administered to a wound in an amount effective to seal the wound;
b) administered to seal dura, lung membrane, peritoneum, gastrointestinal, endothelium, or burned tissue; or
c) administered to a tissue surface to form a barrier.

14. A kit comprising a dosage unit of the composition of any of claims 1-9.

15. The kit of claim 14, wherein the dosage unit:
a) comprises a container for dry components and a container for liquid components, which mixed together form the nanoglue; or
b) is a dropper bottle or a dual barrel syringe with mixing tip or an applicator.

16. The composition of any one of claims 1-9, for use in a method of therapy or surgery.

17. The composition of any one of claims 1-9, for use in healing an eye of an individual in need thereof, wherein upon use, the dendrimers and bioadhesive polymers are photocrosslinked within the eye of the individual.

18. The composition for use according to claim 17, wherein the use is for healing a corneal wound or corneal incision, optionally wherein the composition is for use at the corneal surface of the eye.

19. The composition for use according to claim 18, wherein the corneal wound or corneal incision has arisen from a surgical procedure, optionally wherein the surgical procedure is laser-assisted in situ keratomileusis.

## Patentansprüche

1. Zusammensetzung, die umfasst:
Poly(amidoamin)-(PAMAM)-Dendrimere der Generation 2-10, die eine oder mehrere endständige Hydroxylgruppen und eine oder mehrere photovernetzbare Gruppen umfassen;
bioadhäsive Polymere, die eine oder mehrere photovernetzbare Gruppen umfassen; und
einen Photoinitiator;
wobei die Dendrimere und die bioadhäsiven Polymere über ihre jeweiligen photovernetzbaren Gruppen miteinander vernetzt werden, wenn sie einem oder mehreren externen Reizen ausgesetzt werden, um einen Nanoglue in einem Gewebe zu bilden, und
wobei der externe Reiz ultraviolette Strahlung, kobaltblaues Licht, Argonlaser oder sichtbares Licht ist.

2. Zusammensetzung nach Anspruch 1, wobei die Zusammensetzung ferner ein therapeutisches, prophylaktisches oder diagnostisches Mittel umfasst, das mit den Dendrimeren konjugiert ist.

3. Zusammensetzung nach Anspruch 2, wobei die Dendrimere mit einem Mittel konjugiert sind, das ausgewählt ist aus der Gruppe bestehend aus entzündungshemmenden Mitteln, anti-infektiösen Mitteln, Anti-Glaukom-Mitteln, Mitteln, die den intraokularen Druck (IOP) senken, Anti-Angiogenese-Mitteln und Wachstumsfaktoren.

4. Zusammensetzung nach Anspruch 2, wobei die Dendrimere mit einem diagnostischen Mittel konjugiert sind, ausgewählt aus der Gruppe bestehend aus paramagnetischen Molekülen, fluoreszierenden Verbindungen, magnetischen Molekülen, Radionukliden, Röntgenfigursmitteln und Kontrastmitteln.

5. Zusammensetzung nach Anspruch 2, wobei die Dendrimere mit einem therapeutischen Mittel konjugiert sind, ausgewählt aus der Gruppe bestehend aus Dexamethason, Betamethason, Triamcinolon, Prednison, Prednisolon, Methylprednisolon, Cortison, Hydrocortison und anderen Steroiden und nicht-steroidalen entzündungshemmenden Medikamenten (NSAIDS).

6. Zusammensetzung nach Anspruch 1, wobei die Dendrimere Poly(amidoamin)-(PAMAM)-Dendrimere der Generation 2-6 sind; optional wobei die Dendrimere PAM-Dendrimere der Generation 4, 5 oder 6 sind.

7. Zusammensetzung nach Anspruch 1, wobei die bioadhäsiven Polymere ausgewählt sind aus der Gruppe bestehend aus Hyaluronsäure, Chitosan, Alginat, Agarose, Carboxymethylcellulose, Hydroxymethylcellulose, Methylcellulose, Cellulose, Polyalkylenoxid, Poly(acrylsäure), Poly(hydroxyethylmethacrylat), Poly(vinylpyrrolidon), Poly(vinylalkohol), Pullulan und Derivaten davon.

8. Zusammensetzung nach Anspruch 1, wobei die bioadhäsiven Polymere Hyaluronsäure oder Derivate davon sind.

9. Zusammensetzung nach Anspruch 1, wobei die photovernetzbaren Gruppen ausgewählt sind aus Thiolgruppen und Maleimidgruppen; Amingruppen und Aldehydgruppen; Amingruppen und Carboxylatgruppen; Methacrylatgruppen; und Acrylatgruppen.

10. Zusammensetzung zur Verwendung in einem Verfahren zum Versiegeln von Gewebe in einem Individuum, das dies benötigt, wobei das Verfahren umfasst:
Verabreichen der Zusammensetzung nach einem der Ansprüche 1 bis 9 an ein Gewebe des bedürftigen Individuums; und
Vernetzen der Dendrimere und bioadhäsiven Polymere innerhalb des Gewebes durch Aussetzen an einen oder mehrere externe Reize, um einen Nanoglue zu bilden,
wobei der externe Reiz ultraviolette Strahlung, kobaltblaues Licht, Argonlaser oder sichtbares Licht ist.

11. Zusammensetzung zur Verwendung nach Anspruch 10, wobei die Zusammensetzung durch Injektion verabreicht wird.

12. Zusammensetzung zur Verwendung nach Anspruch 10, wobei die Zusammensetzung auf die Oberfläche oder das Kompartiment eines Auges (subkonjunktival, intrakameral, supra-choroidal, topisch, intravitreal oder subretinal) oder auf angrenzendes Gewebe verabreicht wird; optional
wobei die Zusammensetzung subkonjunktival als Gel zur anhaltenden Behandlung von Augen-/Hornhautentzündungen verabreicht wird; ferner optional wobei das Verfahren zur Behandlung von Alkali-/Säurebrennen, trockenem Auge, Hornhautentzündung, Hornhautinfektionen, Iritis oder Uveitis dient.

13. Zusammensetzung zur Verwendung nach Anspruch 10, wobei die Zusammensetzung:
a) auf eine Wunde in einer Menge verabreicht wird, die wirksam ist, um die Wunde zu versiegeln;
b) zum Versiegeln von Dura, Lungenmembran, Peritoneum, Magen-Darm-Trakt, Endothel oder verbranntem Gewebe verabreicht wird; oder
c) auf eine Gewebeoberfläche verabreicht wird, um eine Barriere zu bilden.

14. Kit, das eine Dosierungseinheit der Zusammensetzung nach einem der Ansprüche 1-9 umfasst.

15. Kit nach Anspruch 14, wobei die Dosierungseinheit:
a) einen Behälter für trockene Komponenten und einen Behälter für flüssige Komponenten umfasst, die zusammen gemischt den Nanoglue bilden; oder
b) eine Tropfflasche oder eine Doppelzylinder-Spritze mit Mischspitze oder einem Applikator ist.

16. Zusammensetzung nach einem der Ansprüche 1-9 zur Verwendung in einem therapeutischen oder chirurgischen Verfahren.

17. Zusammensetzung nach einem der Ansprüche 1-9 zur Verwendung bei der Heilung eines Auges eines Individuums, das dies benötigt, wobei bei der Verwendung die Dendrimere und bioadhäsiven Polymere im Auge des Individuums photovernetzt werden.

18. Zusammensetzung zur Verwendung nach Anspruch 17, wobei die Verwendung zur Heilung einer Hornhautwunde oder eines Hornhautschnittes erfolgt, optional wobei die Zusammensetzung zur Verwendung an der Hornhautoberfläche des Auges bestimmt ist.

19. Zusammensetzung zur Verwendung nach Anspruch 18, wobei die Hornhautwunde oder der Hornhautschnitt durch einen chirurgischen Eingriff entstanden ist, optional wobei es sich bei dem chirurgischen Eingriff um eine laserunterstützte In-situ-Keratomileusis handelt.

## Revendications

1. Composition, comprenant :
des dendrimères poly(amidoamine) (PAMAM) générations 2-10 comprenant un ou plusieurs groupes hydroxyle terminaux et un ou plusieurs groupes photoréticulables ;
des polymères bioadhésifs comprenant un ou plusieurs groupes photoréticulables ; et
un photoinitiateur ;
dans laquelle les dendrimères et les polymères bioadhésifs sont réticulés les uns avec les autres par le biais de leurs groupes photoréticulables respectifs lors de l'exposition à un ou à plusieurs stimuli externes pour former une nanocolle à l'intérieur d'un tissu, et
dans laquelle les stimuli externes sont un rayonnement ultraviolet, de la lumière bleue cobalt, un laser argon, ou de la lumière visible.

2. Composition de la revendication 1, dans laquelle la composition comprend en outre un agent thérapeutique, prophylactique, ou diagnostique conjugué avec les dendrimères.

3. Composition de la revendication 2, dans laquelle les dendrimères sont conjugués avec un agent sélectionné parmi le groupe constitué de : médicaments anti-inflammatoires, agents anti-infectieux, agents anti-glaucomes, agents qui réduisent la pression intraoculaire (Intra-Ocular Pressure, IOP), agents anti-angiogénique, et facteurs de croissance.

4. Composition de la revendication 2, dans laquelle les dendrimères sont conjugués avec un agent diagnostique sélectionné parmi le groupe constitué de : molécules paramagnétiques, composés fluorescents, molécules magnétiques, radionucléides, agents d'imagerie par rayons X, et produits de contraste.

5. Composition de la revendication 2, dans laquelle les dendrimères sont conjugués avec un agent thérapeutique sélectionné parmi le groupe constitué de : dexaméthasone, bêtaméthasone, triamcinolone, prednisone, prednisolone, méthylprednisolone, cortisone, hydrocortisone, et autres stéroïdes et médicaments anti-inflammatoires non-stéroïdes (Non-Steroidal Anti-Inflammatory Drugs, NSAIDS).

6. Composition de la revendication 1, dans laquelle les dendrimères sont des dendrimères poly(amidoamine) (PAMAM) génération 2-6 ; facultativement dans laquelle les dendrimères sont des dendrimères PAMAM génération 4, 5 ou 6.

7. Composition de la revendication 1, dans laquelle les polymères bioadhésifs sont sélectionnés parmi le groupe constitué de : acide hyaluronique, chitosane, alginate, agarose, carboxyméthylcellulose, hydroxyméthylcellulose, méthylcellulose, cellulose, oxyde de polyalkylène, poly(acide acrylique), poly(méthacrylate d'hydroxyéthyle), poly(vinylpyrrolidone), poly(alcool vinylique), pullulane, et dérivés de ceux-ci.

8. Composition de la revendication 1, dans laquelle les polymères bioadhésifs sont de l'acide hyaluronique ou des dérivés de celui-ci.

9. Composition de la revendication 1, dans laquelle les groupes photoréticulables sont sélectionnés parmi des groupes thiol et des groupes maléimide ; des groupes amine et groupes aldéhyde ; des groupes amine et de groupes carboxylate ; des groupes méthacrylate ; et des groupes acrylate.

10. Composition pour utilisation dans une méthode de collage de tissu chez un individu en ayant besoin, le procédé comprenant :
l'administration à un tissu de l'individu en ayant besoin de la composition de quelconques des revendications 1 à 9 ; et
la réticulation des dendrimères et polymères bioadhésifs à l'intérieur du tissu par exposition à l'un ou aux plusieurs stimuli externes pour former une nanocolle,
dans laquelle les stimuli externes sont un rayonnement ultraviolet, de la lumière bleue de cobalt, un laser argon, ou de la lumière visible.

11. Composition pour utilisation selon la revendication 10, dans laquelle la composition est administrée par injection.

12. Composition pour utilisation selon la revendication 10, dans laquelle la composition est administrée à la surface ou un compartiment d'un œil (sous-conjectival, intracaméral, suprachoroïdien, topique, intravitréen, ou sous-rétinien), ou tissu adjacent ; facultativement
dans laquelle la composition est administrée de façon sous-conjectivale sous forme de gel pour le traitement prolongé d'inflammation oculaire/cornéenne ; en outre facultativement
dans laquelle la méthode est pour le traitement de brûlure alcalines/acides, d'œil sec, d'inflammation cornéenne, d'infections cornéennes, d'iritis, ou d'uvéite.

13. Composition pour use selon la revendication 10, dans laquelle la composition est :
a) administrée à une lésion en une quantité efficace pour coller la lésion ;
b) administrée pour coller la dure-mère, la membrane pulmonaire, le péritoine, un tissu gastrointestinal, l'endothélium, ou un tissu brûlé ; ou
c) administrée à une surface de tissu pour former une barrière.

14. Kit, comprenant une unité de dosage de la composition de quelconques des revendications 1 à 9.

15. Kit de la revendication 14, dans lequel l'unité de dosage :
a) comprend un contenant pour des composants secs et un contenant pour des composants liquides, qui mélangés ensemble forment la nanocolle ; ou
b) est une bouteille compte-gouttes ou une seringue deux corps avec un embout mélangeur ou un applicateur.

16. Composition de l'une quelconque des revendications 1 à 9, pour utilisation dans une méthode de thérapie ou de chirurgie.

17. Composition de l'une quelconque des revendications 1 à 9, pour utilisation dans la guérison d'un œil d'un individu en ayant besoin, dans laquelle, lors de l'utilisation, les dendrimères et polymères bioadhésifs sont photoréticulés à l'intérieur de l'œil de l'individu.

18. Composition pour utilisation selon la revendication 17, dans laquelle l'utilisation est pour la guérison de lésion cornéenne ou d'incision cornéenne, facultativement dans laquelle la composition est pour l'utilisation sur la surface cornéenne de l'œil.

19. Composition pour utilisation selon la revendication 18, dans laquelle la lésion cornéenne ou l'incision cornéenne résulte d'une procédure chirurgicale, facultativement dans laquelle la procédure chirurgicale est un kératomileusis in situ assisté par laser.
